# EUROPEAN PATENT APPLICATION

(11) **EP 1 308 781 A2**
(43) Date of publication of application: **07.05.2003**
(21) Application number: 02256995.8
(22) Date of filing: 04.10.2002
(51) Int. Cl.: G03F 7/004, C07D 333/46, C07D 335/02, C07D 333/48

(54) **Cyclic sulfonium and sulfoxonium photoacid generators and photoresists containing them**

(30) Priority: 05.10.2001 US 327499 P
(71) Applicant: Shipley Co. L.L.C., Marlborough, MA 01752 (US)
(72) Inventor: Cameron, James F., Cambridge, Massachusetts 02139 (US); Pohlers, Gerhard, Newton, Massachusetts 02458 (US); Suzuki, Yasuhiro, Cambridge, Massachusetts 02139 (US)
(74) Representative: Kent, Venetia Katherine

(57) **Abstract**

New photoacid generator compounds ("PAGs") are provided and photoresist compositions that comprise such compounds. In particular, cyclic sulfonium and sulfoxonium PAGs are provided. PAGs of the invention are particularly useful as photoactive components of photoresists imaged at short wavelengths such as 248 nm, 193 nm and 157 run.

## Description

The present application claims the benefit of U.S. provisional application number 60/327,499 filed October 5, 2001, which is incorporated herein by reference in its entirety.

### BACKGROUND OF THE INVENTION

### 1. Field of the Invention

This invention relates to new photoacid generator compounds ("PAGs") and photoresist compositions that comprise such compounds. In particular, the invention relates to sulfonium and sulfoxium photoacid generator compounds wherein the sulfonium (i.e. S⁺) or sulfoxonium (i.e. S(O)⁺) is a ring member, i.e. cyclic sulfonium and sulfoxonium PAGs. PAGs of the invention are preferably employed in resists imaged at short wavelengths, such as sub-300 nm and sub-200 nm, e.g. 248 nm, 193 nm and 157 nm, and other higher energy imaging sources such as EUV, X-ray and the like.

### 2. Background

Photoresists are photosensitive films for transfer of images to a substrate. They form negative or positive images. After coating a photoresist on a substrate, the coating is exposed through a patterned photomask to a source of activating energy such as ultraviolet light to form a latent image in the photoresist coating. The photomask has areas opaque and transparent to activating radiation that define an image desired to be transferred to the underlying substrate. A relief image is provided by development of the latent image pattern in the resist coating. The use of photoresists is generally described, for example, by Deforest, Photoresist Materials and Processes, McGraw Hill Book Company, New York (1975), and by Moreau, Semiconductor Lithography, Principals, Practices and Materials, Plenum Press, New York (1988).

Known photoresists can provide features having resolution and size sufficient for many existing commercial applications. However for many other applications, the need exists for new photoresists that can provide highly resolved images of submicron dimension.

Various attempts have been made to alter the make-up of photoresist compositions to improve performance of functional properties. Among other things, a variety of photoactive compounds have been reported for use in photoresist compositions. See, e.g., U.S. Patent 4,450,360 and European Application 615163.

More recently, certain "chemically amplified" photoresist compositions have been reported. Such photoresists may be negative-acting or positive-acting and rely on multiple crosslinking events (in the case of a negative-acting resist) or deprotection reactions (in the case of a positive-acting resist) per unit of photogenerated acid. In other words, the photogenerated acid acts catalytically. In the case of positive chemically amplified resists, certain cationic photoinitiators have been used to induce cleavage of certain "blocking" groups pendant from a photoresist binder, or cleavage of certain groups that comprise a photoresist binder backbone. See, for example, U.S. Patents Nos. 5,075,199; 4,968,851; 4,883,740; 4,810,613; and 4,491,628, and Canadian Patent Application 2,001,384. Upon selective cleavage of the blocking group through exposure of a coating layer of such a resist, a polar functional group is provided, e.g., carboxyl, phenol or imide, which results in different solubility characteristics in exposed and unexposed areas of the resist coating layer.

### SUMMARY OF THE INVENTION

We have now discovered novel cyclic sulfonium and sulfoxonium photoacid generator compounds (PAGs) for use in either positive-acting or negative-acting photoresist compositions. PAGs of the invention contain a sulfonium or sulfoxonium cation that is a ring member of a cyclic group, preferably an alicyclic (non-aromatic) group.

We have found that such PAGs can impart exceptional lithographic performance to a photoresist containing the cyclic PAG. See, for instance, the results set forth in Example 5, which follows.

Particularly preferred are compounds that comprise a sulfur (sulfonium or sulfoxonium) atom as a member of a 4, 5, 6 or 7 membered ring, preferably where each of the other ring members are optionally substituted carbons, and the ring is non-aromatic. Especially preferred are five- and six-membered ring sulfonium and sulfoxonium PAGs, particularly where the sulfonium or sulfoxonium atom is further substituted by an aromatic group such as an optionally substituted carbocyclic aryl group or optionally substituted heteroaromatic group e.g. an optionally substituted phenyl group, optionally substituted naphthyl group, optionally substituted anthracenyl, optionally substituted thienyl group, and other optionally substituted carbocyclic aryl, heteroaromatic and heteroalicyclic groups.

Also preferred are PAGs having a sulfur (sulfonium or sulfoxonium) atom that is a ring member of a multicyclic ring structure, i.e. where two or more ring members of a first ring that contains the sulfonium or sulfoxonium atom form one or more additional cyclic linkages, e.g. to thereby provide a bicyclic sulfonium or sulfoxonium PAG or a tricyclic sulfonioum or sulfoxonium PAG. The additional cyclic linkages of the multicyclic compound may contain optionally substituted saturated carbon atoms or unsaturated carbon atoms (e.g. a keto carbon (-C(=O)- or endocyclic carbon-carbon double bond), or may contain a hetero atom, particularly oxygen, sulfur or sulfinyl or sulfonyl. Such an additional cyclic linkage is suitably linked to adjacent carbon atoms of the ring containing the sulfonium or sulfoxonium atom, or more typically such an additional cyclic linkage is linked to non-adjacent carbon atoms of the ring containing the sulfonium or sulfoxonium atom.

Particularly preferred multicyclic PAG compounds of the invention contain cyclic linkages that contain 5 or 6 atoms as ring members, particularly one or more cyclic linkages that contain 6 atoms as ring members.

Preferred multicyclic sulfonium and sulfoxonium PAGs include those compounds where the sulfonium or sulfoxonium atom is further substituted by an aromatic group such as an optionally substituted carbocyclic aryl or optionally substituted heteroaromatic group e.g. an optionally substituted phenyl group, optionally substituted naphthyl group, optionally substituted anthracenyl, optionally substituted thienyl group, and other optionally substituted carbocyclic aryl, heteroaromatic and heteroalicyclic groups.

Multicyclic PAGs of the invention potentially can exhibit enhanced storage stability (enhanced shelf life) relative to single-ring PAG compounds.

Preferred PAGs of the invention also may have one or more substituents to the alicyclic ring or other substituent of the sulfur (sulfonium or sulfoxonium) atom, e.g. a phenyl, naphthyl, acenaphthyl or other group that has one or more substituents.

Particularly preferred substituents of such substituted PAGs include optionally substituted alkoxy groups such as optionally substituted C₁₋₁₂alkoxy, more typically optionally substituted C₁₋₈alkoxy or C₁₋₆alkoxy. Suitable moieties substituted onto an alkoxy group include e.g. halogen such as fluoro, cyano, nitro and the like.

Especially preferred substituents of such substituted PAGs include optionally substituted photoacid-labile groups.

Surprisingly, we have found that a sulfonium or sulfoxonium PAG of the invention that has a photoacid-labile group moiety linked thereto can impart enhanced adhesion of a photoresist containing the PAG to an underlying substrate, such as a microelectronic wafer substrate (e.g. silicon wafer). Such enhanced adhesion has been particularly exhibited with photoresists imaged with sub-200 nm radiation, particularly 193 nm radiation. Preferred photoacid-labile groups include photoacid-lable esters such as t-butyl esters; acetyl groups such as provided by reaction of a vinyl ether, e.g. an ethyl vinyl ether, and the like. Such photoacid-labile groups may be a substituent of the alicyclic moiety that includes the sulfur (sulfonium or sulfoxonium) moiety, or the photoacid-labile group may be a substituent of the further sulfur atom substituent such as a ring substituent or a phenyl, naphthyl or other moiety. Preferably, the photoacid-labile group is a ring substituent of such an aromatic moiety of the sulfur atom. Especially preferred photoacid-labile substituents of PAGs of the invention have the formula RO(C=O)(CH₂)nO- where R is cyclic or acyclic C₄₋₂₀alkyl and preferably is branched (particularly tertiary carbon linked to oxygen) such as t-butyl, adamantyl, methyladamantyl, ethyladamantyl and the like; and n is an integer of 1 to 8.

Preferably, PAGs of the invention are used in positive-acting or negative-acting chemically amplified photoresists, i.e. negative-acting resist compositions which undergo a photoacid-promoted crosslinking reaction to render exposed regions of a coating layer of the resist less developer soluble than unexposed regions, and positive-acting resist compositions which undergo a photoacid-promoted deprotection reaction of acid labile groups of one or more composition components to render exposed regions of a coating layer of the resist more soluble in an aqueous developer than unexposed regions. Ester groups that contain a tertiary non-cyclic alkyl carbon (e.g. t-butyl) or a tertiary alicyclic carbon (e.g. methyladamantyl) covalently linked to the carboxyl oxygen of the ester are generally preferred photoacid-labile groups of resins employed in photoresists of the invention.

As discussed above, preferred imaging wavelengths of photoresists of the invention include sub-300 nm wavelengths e.g. 248 nm, and sub-200 nm wavelengths e.g. 193 nm and 157 nm.

Particularly preferred photoresists of the invention contain an imaging-effective amount of one or more cyclic sulfonium or sulfoxonium PAGs as disclosed herein and a resin that is selected from the group of:
1) a phenolic resin that contains acid-labile groups that can provide a chemically amplified positive resist particularly suitable for imaging at 248 nm. Particularly preferred resins of this class include: i) polymers that contain polymerized units of a vinyl phenol and an alkyl acrylate, where the polymerized alkyl acrylate units can undergo a deblocking reaction in the presence of photoacid. Exemplary alkyl acrylates that can undergo a photoacid-induced deblocking reaction include e.g. t-butyl acrylate, t-butyl methacrylate, methyladamantyl acrylate, methyl adamantyl methacrylate, and other non-cyclic alkyl and alicyclic acrylates that can undergo a photoacid-induced reaction, such as polymers in U.S. Patents 6,042,997 and 5,492,793, incorporated herein by reference; ii) polymers that contain polymerized units of a vinyl phenol, an optionally substituted vinyl phenyl (e.g. styrene) that does not contain a hydroxy or carboxy ring substituent, and an alkyl acrylate such as those deblocking groups described with polymers i) above, such as polymers described in U.S. Patent 6,042,997, incorporated herein by reference; and iii) polymers that contain repeat units that comprise an acetal or ketal moiety that will react with photoacid, and optionally aromatic repeat units such as phenyl or phenolic groups; such polymers have been described in U.S. Patents 5,929,176 and 6,090,526, incorporated herein by reference, as well as blends of i) and/or ii) and/or iii);
2) a resin that is substantially or completely free of phenyl or other aromatic groups that can provide a chemically amplified positive resist particularly suitable for imaging at sub-200 nm wavelengths such as 193 nm. Particularly preferred resins of this class include: i) polymers that contain polymerized units of a non-aromatic cyclic olefin (endocyclic double bond) such as an optionally substituted norbornene, such as polymers described in U.S. Patents 5,843,624, and 6,048,664, incorporated herein by reference; ii) polymers that contain alkyl acrylate units such as e.g. t-butyl acrylate, t-butyl methacrylate, methyladamantyl acrylate, methyl adamantyl methacrylate, and other non-cyclic alkyl and alicyclic acrylates; such polymers have been described in U.S. Patent 6,057,083; European Published Applications EP01008913A1 and EP00930542A1; and U.S. pending Patent Application No. 09/143,462, all incorporated herein by reference, and iii) polymers that contain polymerized anhydride units, particularly polymerized maleic anhydride and/or itaconic anhydride units, such as disclosed in European Published Application EP01008913A1 and U.S. Patent 6,048,662, both incorporated herein by reference, as well as blends of i) and/or ii) and/or iii);
3) a resin that contains repeat units that contain a hetero atom, particularly oxygen and/or sulfur (but other than an anhydride, i.e. the unit does not contain a keto ring atom), and preferable are substantially or completely free of any aromatic units. Preferably, the heteroalicyclic unit is fused to the resin backbone, and further preferred is where the resin comprises a fused carbon alicyclic unit such as provided by polymerization of a norborene group and/or an anhydride unit such as provided by polymerization of a maleic anhydride or itaconic anhydride. Such resins are disclosed in PCT/US01/14914 and U.S. application number 09/567,634.
4) a resin that contains fluorine substitution (fluoropolymer), e.g. as may be provided by polymerization of tetrafluoroethylene, a fluorinated aromatic group such as fluoro-styrene compound, compounds that comprise a hexafluoroalcohol moiety, and the like. Examples of such resins are disclosed e.g. in PCT/US99/21912.

Resists of the invention also may comprise a mixture of distinct PAGs, typically a mixture of 2 or 3 different PAGs, more typically a mixture that consists of a total of 2 distinct PAGs. At least one PAG of the mixture will be a cyclic sulfonium or sulfoxonium PAG of the invention. The other PAG(s) of the mixture also may be a cyclic sulfonium or sulfoxonium PAG of the invention, or may be another type of PAG, including another onium compound such as an iodonium or sulfonium compound, or other non-ionic compound, preferably without any aromatic content such as an imidosulfonate PAG compound, a diazodisulfone, a disulfone PAG, and the like. Photoresists that contain such PAG mixtures can exhibit even further enhanced lithographic performance.

The invention also provide methods for forming relief images of the photoresists of the invention, including methods for forming highly resolved patterned photoresist images (e.g. a patterned line having essentially vertical sidewalls) of sub-quarter micron dimensions or less, such as sub-0.2 or sub-0.1 micron dimensions.

The invention further provides articles of manufacture comprising substrates such as a microelectronic wafer or a flat panel display substrate having coated thereon the photoresists and relief images of the invention.

The invention further comprises methods for synthesis of PAGs of the invention.

The invention also contains specifically preferred photoresist compositions.

Other aspects of the invention are disclosed *infra.*

### DETAILED DESCRIPTION OF THE INVENTION

As discussed above, we now provide cyclic sulfonium and sulfoxonium PAGs that are particularly useful for positive-acting chemically amplified resists, such as resists imaged at 248 nm, 193 nm or 157 nm, and other higher energy imaging sources such as EUV, X-ray and the like.

Preferred PAGs of the invention include sulfonium PAGs of the following Formula I: 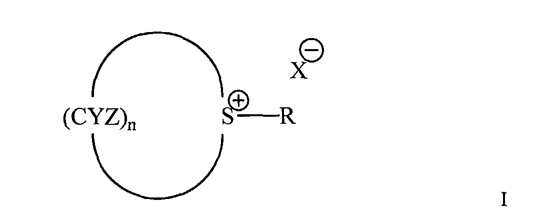 wherein R is a non-hydrogen substituent such as optionally substituted alkyl preferably having 1 to about 20 carbon atoms; optionally substituted alkenyl preferably having 2 to about 20 carbon atoms; optionally substituted alkynyl preferably having 1 to about 20 carbon atoms; optionally substituted carbocyclic aryl such as optionally substituted phenyl particularly pentafluorophenyl and optionally substituted naphthyl and optionally substituted anthracenyl; optionally substituted heteroaromatic or heteroalicyclic particularly optionally substituted thienyl,
or R is a linkage to another PAG moiety, particularly another cyclic sulfonium group, e.g. R is a chemical bond, an optionally substituted alkylene linkage suitably having 1 to about 12 carbon atoms, an optionally substituted alkenylene linkage suitably having 2 to about 12 carbon atoms, an optionally substituted alkynylene linkage suitably having 2 to about 12 carbon atoms, an optionally substituted heteroalkylene linkage suitably having 1 to about 12 carbon atoms, an optionally substituted heteroalkenylene linkage suitably having 2 to about 12 carbon atoms, an optionally substituted heteroalkynylene linkage suitably having 2 to about 12 carbon atoms, an optionally substituted carbocyclic aryl, an optionally substituted aralkyl, an optionally substituted heteroaromatic, an optionally substituted heteroaralkyl, and the like;
Y and Z are each independently hydrogen or a non-hydrogen substituent such as those specified above for R;
or two of Y and/or Z are taken together to form a fused ring (i.e. fused to the depicted cyclic sulfonium ring) that may be a carbon alicyclic group, carbocyclic aryl, heteroalicyclic or heteroaromatic, and preferably is a carbon alicyclic or heteroalicyclic;
n is an integer of from 3 to 8, preferably 4 or 5; and
X is a counter anion,
particularly an organic anion such as a sulfonate e.g. ofthe formula R'SO₃ where R' is suitably optionally substituted alkyl, particularly perfluoroalkyl typically having 1 to about 12 carbon atoms such as triflate and the like; carbocyclic aryl sulfonate such as pentafluorophenylsulfonate and a trifluoromethylbenzene sulfonate particularly 2-trifluoromethyl benzene sulfonate; a carbon alicyclic sulfonate such as camphorsulfonate and the like;
or a carboxylate, e.g. groups of the formula R"COO- where R" is optionally substituted alkyl having 1 to about 18 carbons or optionally substituted aryl such as phenyl and the like, preferred substituents of substituted carboxylate anions include halo, particularly fluoro;
or a sulfonylimide, particularly alkyl sulfonylimides, especially halogenated alkylsulfonylimides such as fluoroalkyl sulfonylimides e.g. perfluoroalkyl sulfonylimides including perfluoroC₁₋₆alkyl sulfonylimides;
or a sulfonylmethide, particularly alkyl sulfonylmethides, especially halogenated alkylsulfonylmethides such as fluoroalkyl sulfonylmethides e.g. perfluoroalkyl sulfonylmethides including perfluoroC₁₋₆alkyl sulfonylmethides.

As mentioned above, generally preferred sulfonium PAGs of the invention contain a ring with 5 or 6 ring members inclusive of the sulfonium atom, such as compounds of the following Formulae IA and IB: 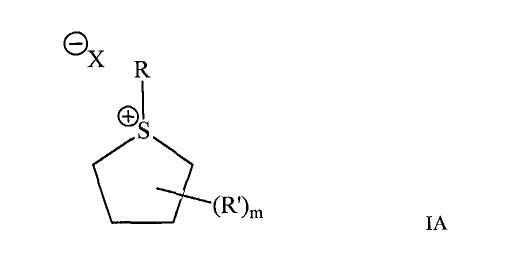 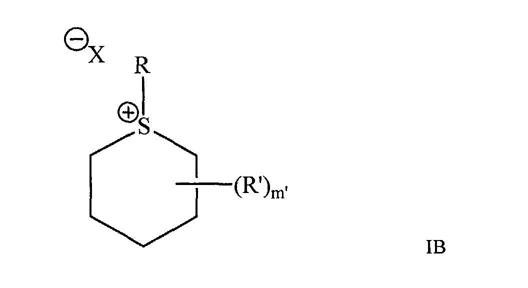 wherein in each of Formulae IA and IB:
R and X are each the same defined in Formula I above;
R¹ is a non-hydrogen substituents the same as defined for R in Formula I above;
m is an integer of from 0 to 8, preferably 0, 1, 2, 3 or 4; m' is an integer of from 0 to 10, preferably 0, 1, 2, 3, 4 or 5.

In the above Formulae I, IA and IB, less preferred, and thus excluded from certain preferred aspects of the invention, are compounds with R being carboxylic aryl particularly unsubstituted phenyl or unsubstituted naphthyl and/or phenyl or naphthyl having one or more hydroxy or blocked hydroxy (e.g. alkoxy such as C₁₋₆alkoxy or C₁₋₄alkoxy such as methoxy, aryloxy such as phenoxy) ring substituents; and/or compounds where a single sulfonium ring carbon atom has two alkyl substituents such as two C₁₋₄alkyl substituents (i.e. in Formula 1, for a single sulfur ring carbon, each of Y and Z is alkyl; and in Formulae IA or IB, on a single sulfur ring carbon atoms, two R¹ substituents are alkyl).

Also preferred are multiple-sulfonium compounds, i.e. where a single compounds contains multiple sulfonium atoms, preferably 2, 3 or 4 sulfonium atoms, particularly a single compound that contains two sulfonium atoms (a bis-sulfonium compound). In particular, preferred are bis-sulfonium compounds of the following Formula II: 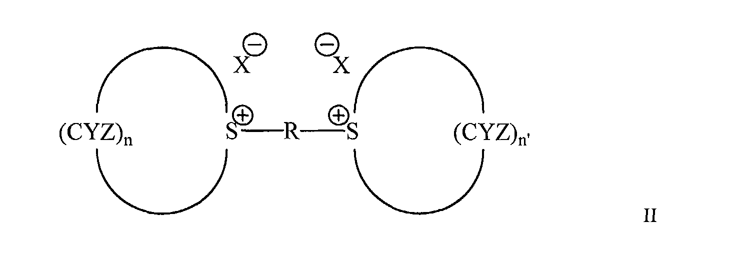 wherein Formula II, R is the same as defined in Formula I above; each Y and each Z are independently the same as defined for Y and Z in Formula I above; n and n' are each independently an integer of from 3 to 8, preferably 4 or 5; and each X is the same or different and are the same as defined for X in Formula I above.

Generally preferred are such bis-compounds that comprise 5- or 6-membered sulfonium groups such as compounds of the following Formulae IIA and IIB: 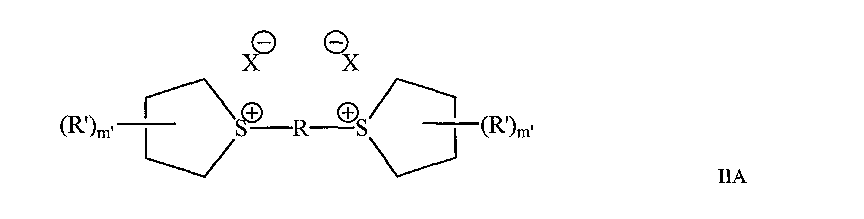 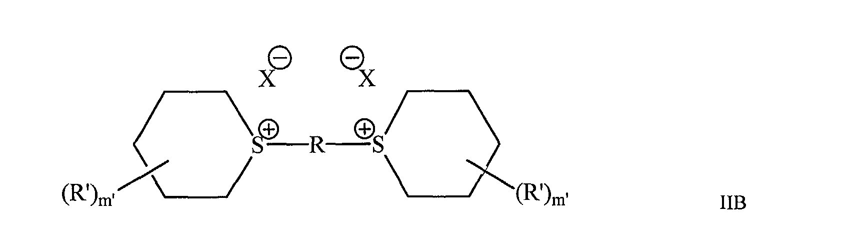 wherein in each of Formulae IIA and IIB:
R, each R¹ and X each are the same defined in Formula IA and IB above;
each m is independently an integer of from 0 to 8, preferably 0, 1, 2, 3, or 4; and each m' is independently an integer of from 0 to 10, preferably 0, 1, 2, 3, 4 or 5.

As discussed above, preferred PAGs of the invention also include sulfoxonium PAGs, such as compounds of the following Formula III: 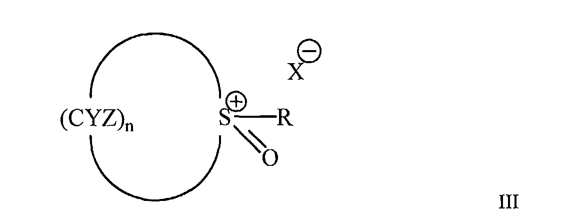 wherein R is a non-hydrogen substituent such as optionally substituted alkyl preferably having 1 to about 20 carbon atoms; optionally substituted alkenyl preferably having 2 to about 20 carbon atoms; optionally substituted alkynyl preferably having 1 to about 20 carbon atoms; optionally substituted carbocyclic aryl such as optionally substituted phenyl particularly pentafluorophenyl and optionally substituted naphthyl and optionally substituted anthracenyl; optionally substituted heteroaromatic or heteroalicyclic particularly optionally substituted thienyl,
or R is a linkage to another PAG moiety, particularly another cyclic sulfoxonium group or a cyclic sulfonium group such as those of Formulae I, IA, IB or II above, e.g. R is a chemical bond, an optionally substituted alkylene linkage suitably having 1 to about 12 carbon atoms, an optionally substituted alkenylene linkage suitably having 2 to about 12 carbon atoms, an optionally substituted alkynylene linkage suitably having 2 to about 12 carbon atoms, an optionally substituted heteroalkylene linkage suitably having 1 to about 12 carbon atoms, an optionally substituted heteroalkenylene linkage suitably having 2 to about 12 carbon atoms, an optionally substituted heteroalkynylene linkage suitably having 2 to about 12 carbon atoms, an optionally substituted carbocyclic aryl, an optionally substituted aralkyl, an optionally substituted heteroaromatic, an optionally substituted heteroaralkyl, and the like;
Y and Z are each independently hydrogen or a non-hydrogen substituent such as those specified above for R;
or two of Y and/or Z are taken together to form a fused ring (i.e. fused to the depicted cyclic sulfoxonium ring) that may be a carbon alicyclic group, carbocyclic aryl, heteroalicyclic or heteroaromatic, and preferably is a carbon alicyclic or heteroalicyclic;
n is an integer of from 3 to 8, preferably 4 or 5; and
X is a counter anion,
particularly an organic anion such as a sulfonate e.g. of the formula R'SO₃ where R' is suitably optionally substituted alkyl, particularly perfluoroalkyl typically having 1 to about 12 carbon atoms such as triflate and the like; carbocyclic aryl sulfonate such as pentafluorophenylsulfonate and a trifluoromethylbenzene sulfonate particularly 2-trifluormethyl benzene sulfonate; a carbon alicyclic sulfonate such as camphorsulfonate and the like;
or a carboxylate, e.g. groups of the formula R"COO- where R" is optionally substituted alkyl having 1 to about 18 carbons or optionally substituted aryl such as phenyl and the like, preferred substituents of substituted carboxylate anions include halo, particularly fluoro;
or a sulfonylimide, particularly alkyl sulfonylimides, especially halogenated alkylsulfonylimides such as fluoroalkyl sulfonylimides e.g. perfluoroalkyl sulfonylimides including perfluoroC₁₋₆alkyl sulfonylimides;
or a sulfonylmethide, particularly alkyl sulfonylmethides, especially halogenated alkylsulfonylmethides such as fluoroalkyl sulfonylmethides e.g. perfluoroalkyl sulfonylmethides including perfluoroC₁₋₆alkyl sulfonylmethides.

As mentioned above, generally preferred sulfoxonium PAGs of the invention contain a ring with 5 or 6 ring members inclusive of the sulfoxonium atom, such as compounds of the following Formulae IIIA and IIIB: 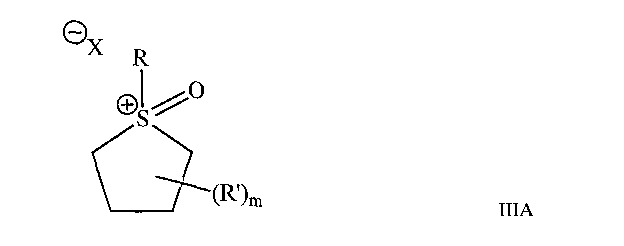 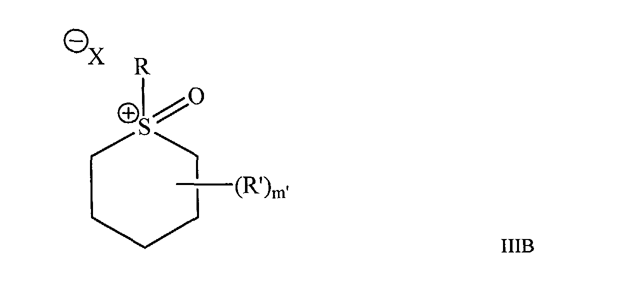 wherein in each of Formulae IIIA and IIIB:
R and X are each the same defined in Formula III above;
R¹ is a non-hydrogen substituents the same as defined for R in Formula above;
m is an integer of from 0 to 8, preferably 0, 1, 2, 3 or 4; m' is an integer of from 0 to 10, preferably 0, 1, 2, 3, 4 or 5.

Also preferred are multiple-sulfoxonium compounds, i.e. where a single compounds contains multiple sulfoxonium atoms, preferably 2, 3 or 4 sulfoxonium atoms, particularly a single compound that contains two sulfoxonium atoms (a bis-sulfoxonium compound). In particular, preferred are bis-sulfoxonium compounds of the following Formula IV: 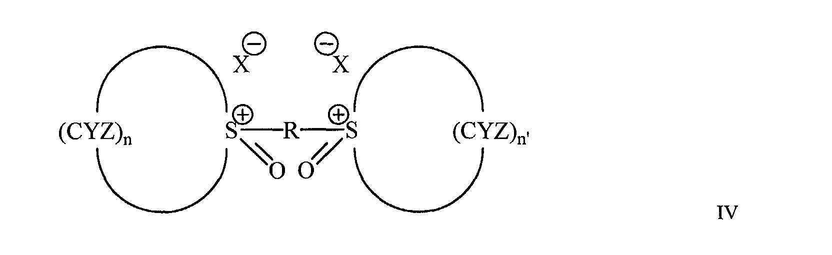 wherein Formula IV, R is the same as defined in Formula III above; each Y and each Z are independently the same as defined for Y and Z in Formula III above; n and n' are each independently in integer of from 3 to 8, preferably 4 or 5; and each X is the same or different and are the same as defined for X in Formula III above.

Generally preferred are such bis-compounds that comprise 5- or 6-membered sulfonium groups such as compounds of the following Formulae IVA and IVB: 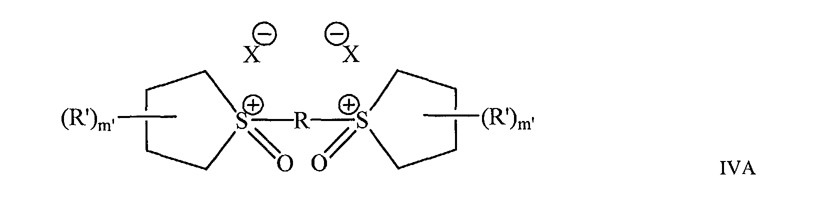 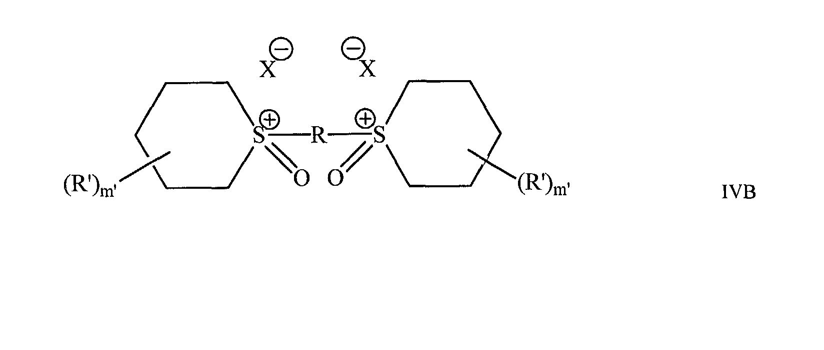 wherein in each of Formulae IVA and IVB:
R, each R¹ and X each are the same defined in Formula III above;
each m is independently an integer of from 0 to 8, preferably 0, 1, 2, 3, 4 or 5 4; and each m' is independently an integer of from 0 to 10, preferably 0, 1, 2, 3, 4 or 5.

As discussed above, multicyclic compounds also are suitable, particularly PAGs having a sulfur (sulfonium or sulfoxonium) atom that is a ring member of a multicyclic ring structure, i.e. where two or more ring members of a first ring that contains the sulfonium or sulfoxonium atom form one or more additional cyclic linkages.

Particularly preferred multicyclic compound include compounds of Formulae I and III as those formulae are defined above, but where Y and Z groups on adjacent or non-ajacent ring atoms are taken together to form a further cyclic linkage. Such additional cyclic linkages will typically contain, in addition to the ring atoms of the sulfonium or sulfoxonium ring, 2 to 6 additional carbon or hetero (particularly O, S, S(O) or S(O)₂), more typically 3, 4 or 5 atoms in addition to the ring atoms of the sulfonium or sulfoxonium ring.

As discussed above, preferred PAGs of the invention include those that have further substitution by one or more alkoxy groups and/or one or more photoacid-labile groups such as a photoacid-labile ester or acetal moiety.

Particularly preferred PAGs include those of the following Formula V: 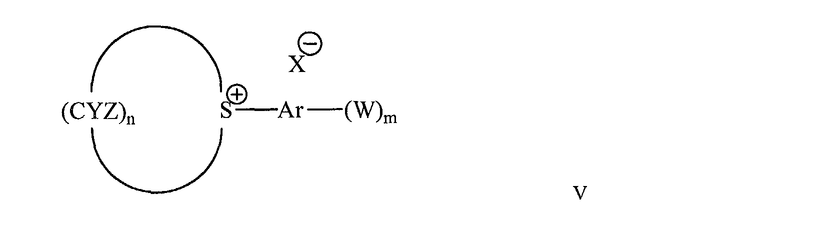 wherein X, Y, Z and n are the same as defined in the above formulae, and preferably n is 4 or 5 (to provide a 5- or 6-membered ring);
Ar is an aryl group, preferably a carbocyclic aryl such as phenyl, naphthyl or acenaphthyl, which may be optionally substituted by non-hydrogen substituents other than W such as a halogen (F, Cl, Br or I), Cl-8alkyl, nitro, and the like;
each W is an optionally substituted alkoxy or photoacid-labile ring substituent of the Ar group;
and m is an integer of from 1 (single W group) to 5, and preferably m is an integer of 1, 2 or 3 (total of 1 to 3 W groups).

Preferred alkoxy W groups of Formula V include optionally substituted C₁₋₈alkoxy, particularly C₁₋₆alkoxy. Preferred photoacid-labile W groups of Formula V include photoacid-labile ester and acetal groups, including photoacid-labile esters of the formula RO(C=O)(CH₂)ₚO- where R is cyclic or acyclic C₄₋₂₀alkyl and preferably is branched (particularly tertiary carbon linked to oxygen) such as t-butyl, adamantyl, methyladamantyl, ethyladamantyl and the like; and p is an integer of 1 to 8, and preferably p is 1, 2, 3 or 4.

Phenyl is a generally preferred Ar group of PAGs of Formula V above, such as PAGs of the following Formula VA: 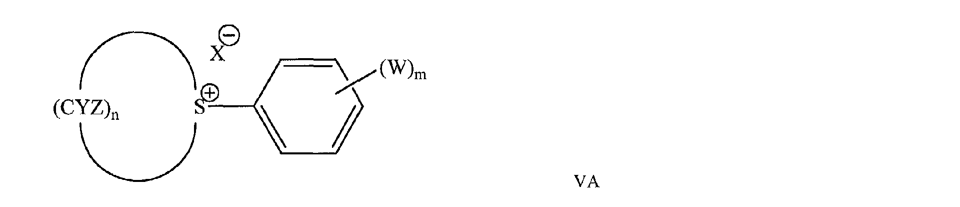 wherein in Formula VA, W X, Y, Z, m, and n are the same as defined in Formula V above, and preferably n is 4 or 5 (to provide a 5- or 6-membered ring).

Such substituted sulfoxonium PAGs also are particularly preferred, such as compounds of the following Formula VI: 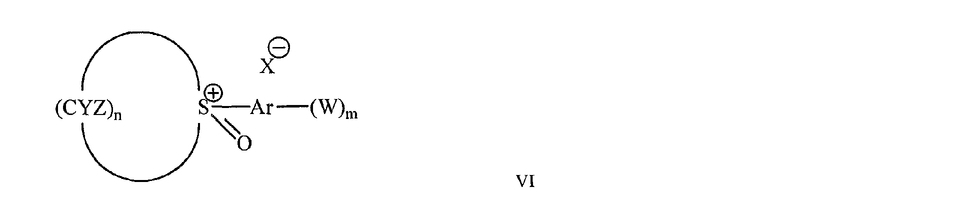 wherein X, Y, Z and n are the same as defined in the above formulae, and preferably n is 4 or 5 (to provide a 5- or 6-membered ring);
Ar is an aryl group, preferably a carbocyclic aryl such as phenyl, naphthyl or acenaphthyl, which may be optionally substituted by non-hydrogen substituents other than W such as a halogen (F, Cl, Br or I), C₁₋₈alkyl, nitro, and the like;
each W is an optionally substituted alkoxy or photoacid-labile ring substituent of the Ar group;
and m is an integer of from 1 (single W group) to 5, and preferably m is an integer of 1, 2 or 3 (total of 1 to 3 W groups).

Preferred alkoxy W groups of Formula VI include optionally substituted C₁₋₈alkoxy, particularly C₁₋₆alkoxy. Preferred photoacid-labile W groups of Formula VI include photoacid-labile ester and acetal groups, including photoacid-labile esters of the formula RO(C=O)(CH₂)ₚO- where R is cyclic or acyclic C₄₋₂₀alkyl and preferably is branched (particularly tertiary carbon linked to oxygen) such as t-butyl, adamantyl, methyladamantyl, ethyladamantyl and the like; and p is an integer of 1 to 8, and preferably p is 1, 2, 3 or 4.

Phenyl is a generally preferred Ar group of sulfoxonium PAGs of Formula VI above, such as PAGs of the following Formula VIA: 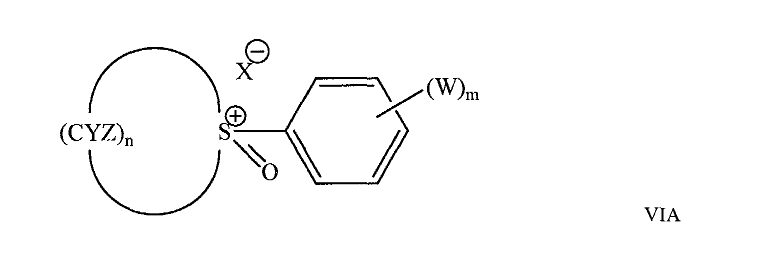 wherein in Formula VIA, W X, Y, Z, m, and n are the same as defined in Formula V above, and preferably n is 4 or 5 (to provide a 5- or 6-membered ring).

Specifically preferred PAGS for use in accordance with the invention include the following compounds 1 through 22, where

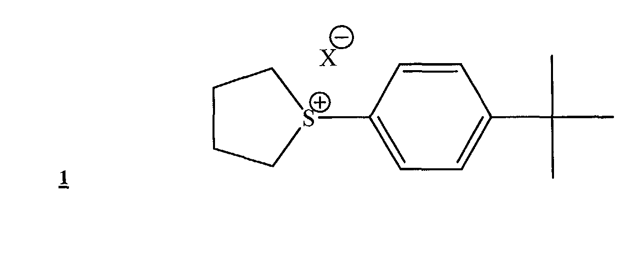

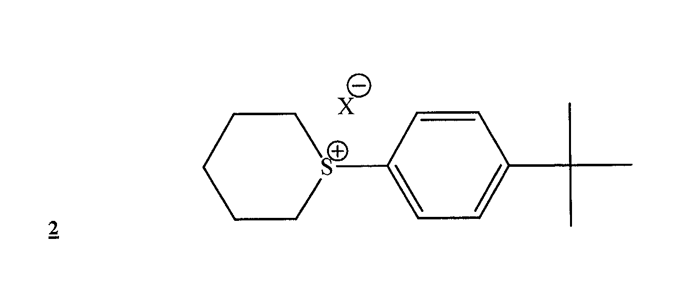

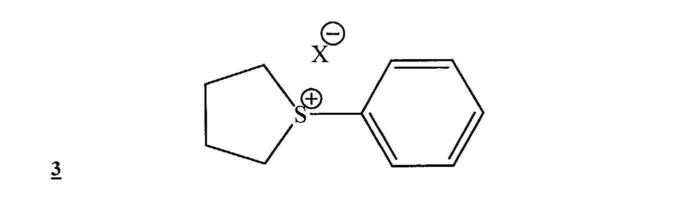

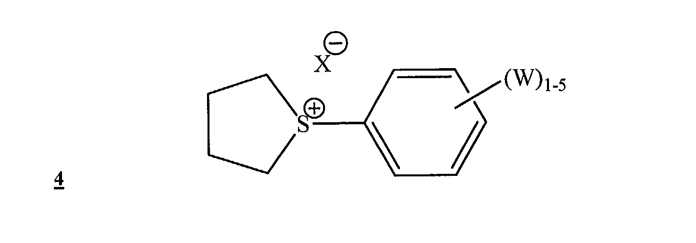

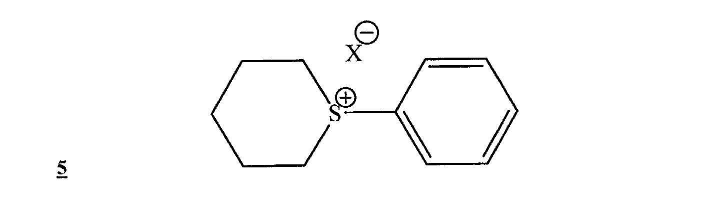

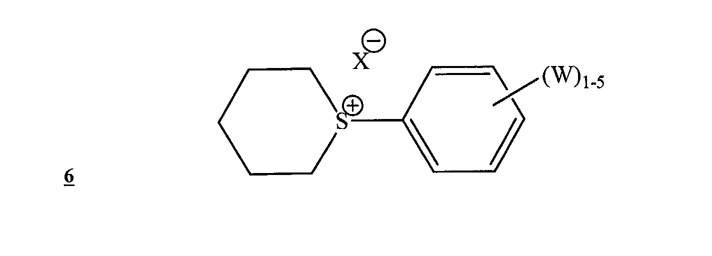

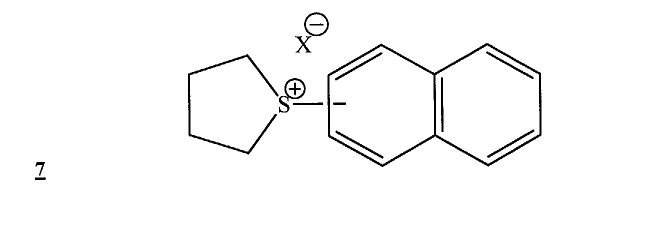

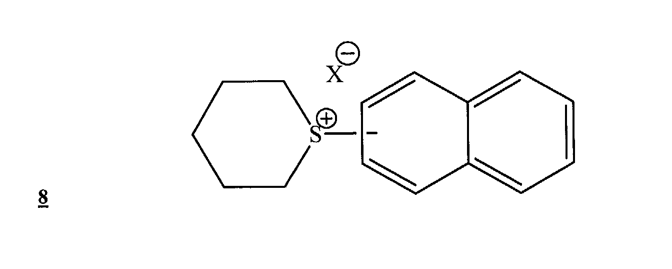

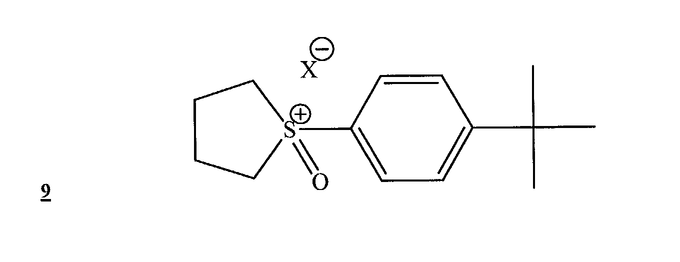

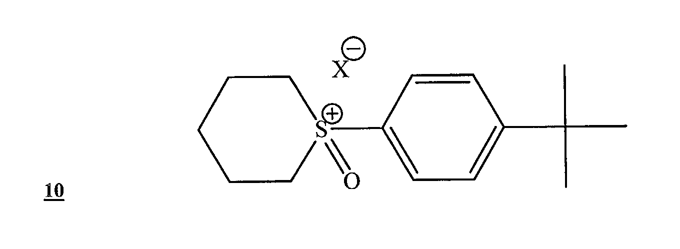

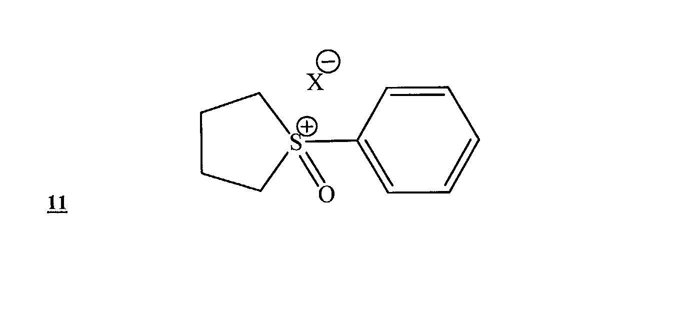

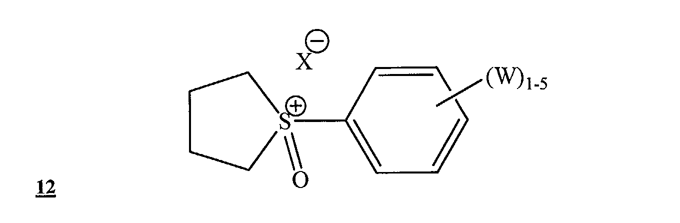

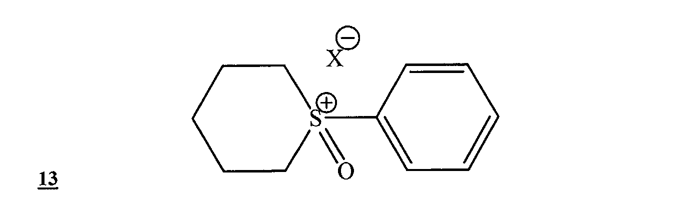

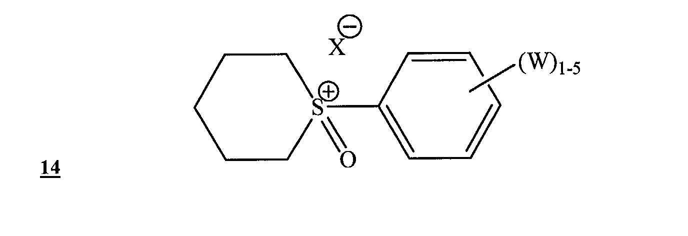

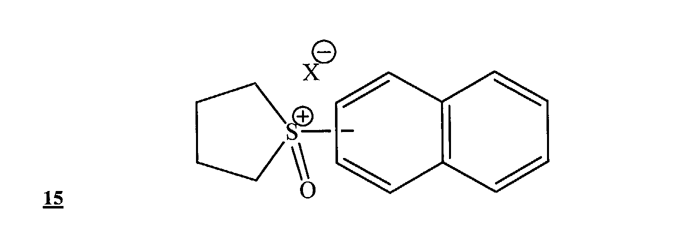

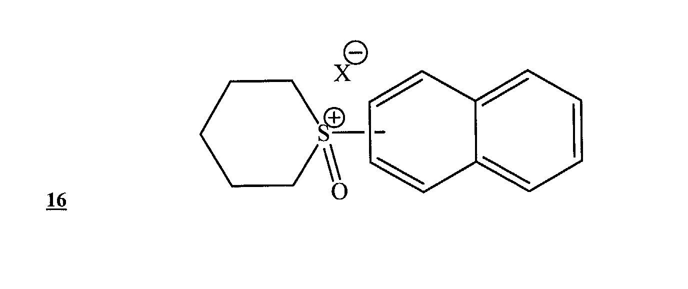

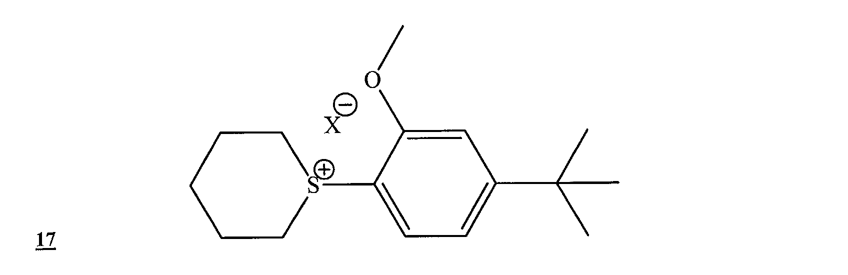

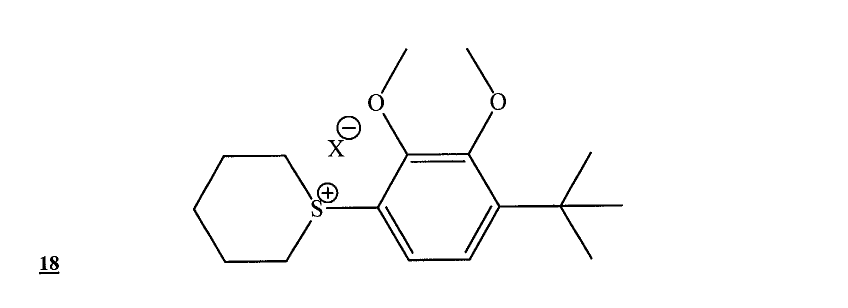

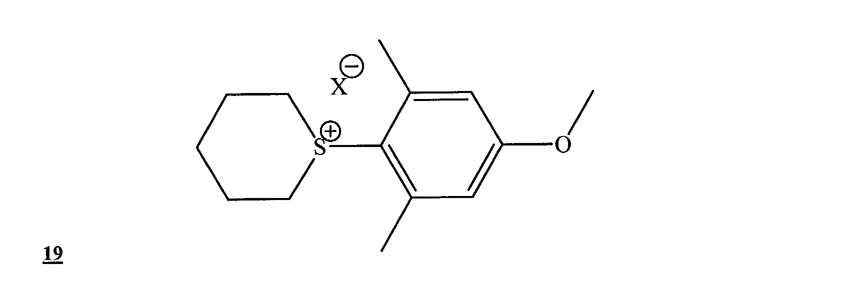

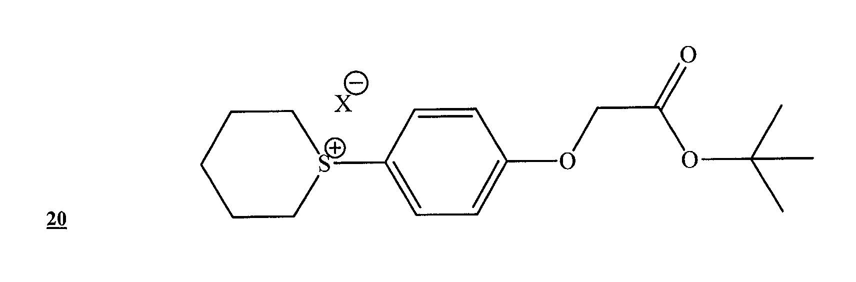

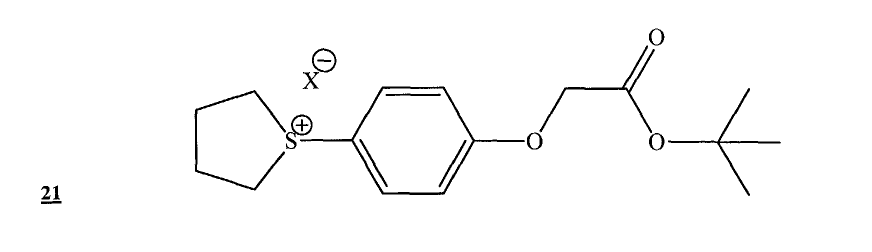

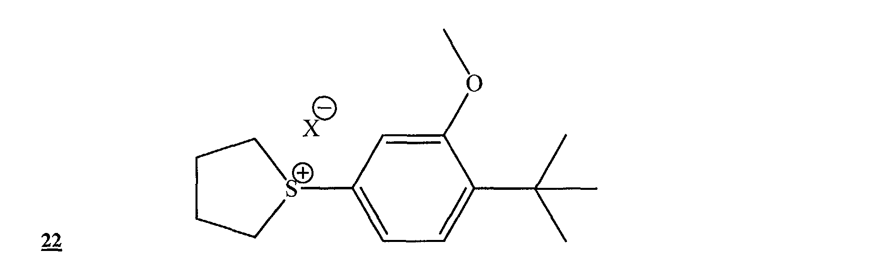

In those compounds 1 through 22 each W is independently hydrogen or optionally substituted alkyl suitably having 1 to about 20 carbon atoms more typically 1 to about 6 carbon atoms, or optionally substituted alkoxy suitable having 1 to about 20 carbon atoms more typically 1 to about 6 carbon atoms; and each X is the same as defined above in Formula I. On any single compound, typically at least one W substituents would be other than hydrogen.

As stated above, various substituent groups of PAGs of the invention may be optionally substituted. Substituted moieties (including substituted R, R', W, X, Y, and Z groups of Formulae I, IA, IB, II, IIA, IIB, III, IIIA, IIIB, IV, IVA, IVB, V, VA, VI, and VIA) are suitably substituted at one or more available positions by, e.g., halogen such as F, Cl Br and/or I, nitro, cyano, sulfono, alkyl including C₁₋₁₆ alkyl with C₁₋₈alkyl being preferred, haloalkyl such as fluoroalkyl (e.g. trifluoromethyl) and perhaloalkyl such as perfluoroC₁₋₄alkyl, alkoxy including C₁₋₁₆alkoxy having one or more oxygen linkages with C₁₋₈alkoxy being preferred, alkenyl including C₂₋₁₂alkenyl with C₂₋₈alkenyl being preferred, alkenyl including C₂₋₁₂alkenyl with C₂₋₈alkynyl being preferred, aryl such as phenyl or naphthyl and substituted aryl such as halo, alkoxy, alkenyl, alkynyl and/or alkyl substituted aryl, preferably having the number of carbon atoms mentioned above for corresponding groups. Preferred substituted aryl groups include substituted phenyl, anthracenyl and naphthyl.

As used herein, the term alkyl, alkenyl and alkynyl unless otherwise modified refers to both cyclic and noncyclic groups, although of course cyclic groups will comprise at least three carbon ring members. Alkenyl and alkynyl groups of compounds of the invention have one or more unsaturated linkages, typically 1 to about 3 or 4 unsaturated linkages. Also, the terms alkenyl and alkynyl as used herein refer to both cyclic and noncyclic groups, although straight or branched noncyclic groups are generally more preferred. Alkoxy groups of PAG compounds of the invention have one or more oxygen linkages, typically 1 to about 5 or 6 oxygen linkages. Suitable alkanoyl groups have one or more carbonyl groups, typically 1 to about 4 or 5 carbonyl groups. Carbocyclic aryl as used herein refers to non-hetero aromatic groups that have 1 to 3 separate or fused rings and 6 to about 18 carbon ring members and may include e.g. phenyl, naphthyl, biphenyl, acenaphthyl, phenanthracyl, and the like. Phenyl and naphthyl are often preferred. Suitable heteroaromatic or heteroaryl groups will have 1 to 3 rings, 3 to 8 ring members in each ring and from 1 to about 3 hetero atoms (N, O or S). Specifically suitable heteroaromatic or heteroaryl groups include e.g. courmarinyl, quinolinyl, pyridyl, pyrazinyl, pyrimdinyl, furyl, pyrrolyl, thienyl, thiazolyl, oxazolyl, imidazolyl, indolyl, benzofuranyl, and benzothiazole.

PAGs of the invention can be readily prepared by a number of synthetic routes.

For instance, a formed cyclic sulfur compound, e.g. an alicyclic sulfide (i.e. sulfur ring member) or alicyclic sulfoxide (i.e. S(O) ring member), reacted with a further reactive compound to provide the tri-substituted cyclic sulfonium compound.

In particular, alicyclic sulfonium compounds may be reacted in the presence of an acid and a phosphoro oxide reagent together with a reactive compound such as an aromatic reagent followed by treatment with a second acid for formation of the sulfonium salt.

A cyclic sulfur compound may be suitably reacted in the presence of a metal catalyst, e.g. a Cu(II) reagent such as copper benzoate, with an iodonium compound to provide a tri-substituted sulfonium, i.e. a type of exchange reaction where the iodonium moiety is substituted to the S atom to form the sulfonium compound. The anion of the iodonium reagent also suitably is transferred to the formed sulfonium compound.

A di-substituted acyclic sulfur compound also can be cyclized to provide an alicyclic sulfonium compound of the invention. For instance, a disubstituted sulfur reagent that comprises a substituent with a suitable leaving group, e.g. an alkyl moiety having a terminal carbon substituted with a halogen (I, Br or Cl), or a sulfonyl ester such as mesyl or tosyl can be cyclized e.g. in the presence of trialkylsilylhalide such as trimethylsilylchloride. The sulfonium salt may be suitably provided e.g. by an exchange reaction, e.g. by treatment with a silver acid salt.

A bis-type sulfonium compound (i.e. a single compound that contains two sulfonium atoms, such as compounds of Formulae II, IIA and IIB above) also may be suitably synthesized by a multiple cyclization reaction.

More particularly, in a first synthetic method of the invention, which provides a condensation-type reaction, i) a sulfoxide reagent, preferably alicyclic sulfoxide such as tetramethylenesulfoxide ((CH₂)₄>S(O)) and ii) a compound reactive with the sulfoxide such as an optionally substituted phenyl or naphthyl compound or other optionally substituted carbocyclic aryl, or optionally substituted heteroalicyclic or heteroaromatic compound such as optionally substituted thienyl, is reacted with phosphorus oxide under acidic conditions e.g. in the presence of an organic acid such as methansulfonic acid or triflic acid or the like or an inorganic acid such as HCl. Suitably the mixture is heated e.g. to about 40°C or greater such as about 50°C or 60°C, with stirring and then quenched with an acidic aqueous solution to provide a cyclic sulfonium compound of the invention. The counter anion of the sulfonium compound suitably may be the same as the acid present in the acidic aqueous quench solution, or the formed salt may be treated with another acid to provide a distinct anion. See Examples 1 and 2 which follow for exemplary preferred reaction conditions and the following Scheme 1, where n is suitably 3, 4, 5, 6 or 7, preferably 4 or 5:

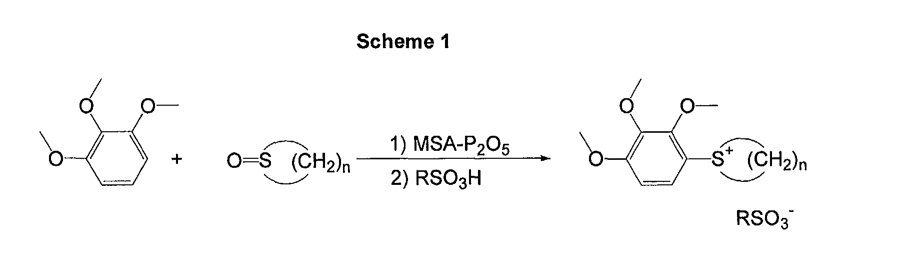

In an iodonium transfer-type reaction, an iodonium compound, having the same or different substituents on the I⁺ atom is reacted with a cyclic sulfur compound such as tetrahydrothiophene in the presence of a suitable catalyst e.g. a Cu(II) compound such as copper benzoate. An inert solvent is typically employed, such as an aromatic solvent e.g. chlorobenzene or xylenes. The reaction is preferably run at elevated temperatures e.g. at least about 60°C, 70°C, 80°C, 90°C, 100°C or 110°C. The cation of the iodonium reagent also suitably is transferred in the reaction to the formed sulfonium compound. See Example 3 which follows for exemplary preferred reaction conditions and the following Scheme 2, where n is suitably 3, 4, 5, 6 or 7, preferably 4 or 5:

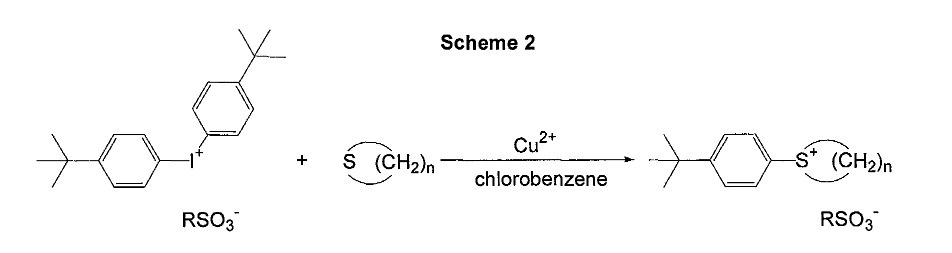

As discussed above, in a cyclization reaction of the invention, a disubstituted thio reagent having at least one substituent with a leaving group is employed, particularly an alkyl group substituted with a leaving group, preferably at a terminal carbon of the alkyl chain. Suitable leaving groups include e.g. halogen such as I, Br or Cl, or a sulfonyl ester e.g. a mesyl or tosyl group. The substituted thio compound then can undergo a cyclization reaction, e.g. by reaction with an alkylsilyl reagent such as trimethylsilyl reagent. The sulfonium salt may be suitably provided e.g. by an exchange reaction, such as by treatment with a silver acid salt. The following Scheme 3 exemplifies such a cyclization reaction, where n is suitably 3, 4, 5, 6 or 7, preferably 4 or 5:

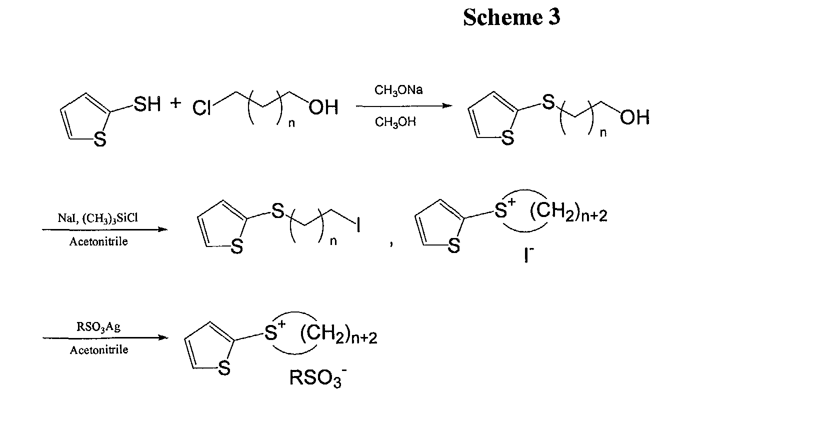

A bis-type sulfonium compound (i.e. a single compound that contains two sulfonium atoms, such as compounds of Formulae II, IIA and/or IIB above) also may be suitably synthesized by a multiple cyclization reaction, in the same general manner as discussed above, but where a compound having multiple thio substituents in employed as a starting reagent. See Example 4 which follows for exemplary conditions for synthesis of a bis-sulfonium compound.

Cyclic sulfoxonium PAG compounds of the invention can be suitably prepared by oxidation of the corresponding cyclic sulfonium compounds, e.g. by treatment of a cyclic sulfonium compound with a peroxide (e.g. H₂O₂) or other suitable oxidant.

Suitable and preferred sulfide reagents used to form sulfonium and sulfoxonium PAGs as detailed above are commercially available and can be readily prepapared. Two preferred sulfide reagents include the following substituted cyclic sulfides, which can be prepared as described below.

### 1. Synthesis of 4,4-dimethylthiopyrane

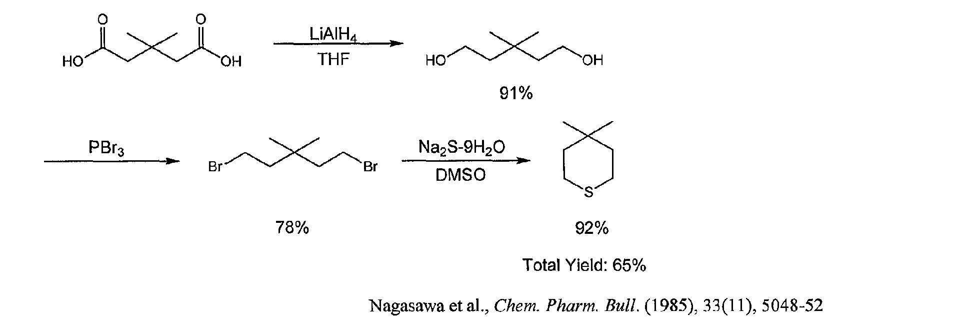

### 2. Synthesis of 4,4-dimethylthiopyrane

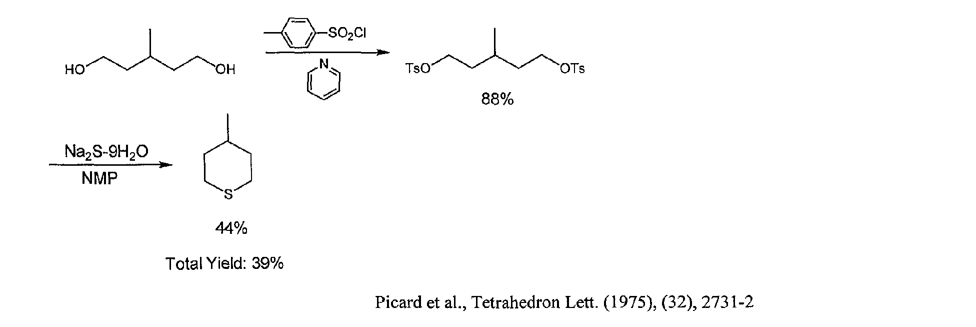

Various substituents of PAGs of the invention can be readily provided. For instance, the starting reagent can include such substituents, or the formed sulfonium or sulfoxonium PAG can be reacted to provide the desired substituent. More particularly, a formed PAG having a phenol or other aryl hydroxy substituent of the sulfur (sulfonium or sulfoxonium) atom can be reacted to provide a photoacid-labile substituent, e.g. a vinyl ether (to provide a photoacid-labile acetal) or chloroacetate such as t-butylchloroacetate (to provide a photoacid-labile ester) can be reacted with an aryl hydroxy moiety such as a phenol suityably under basic conditions to provide the photoacid-labile PAG substituent.

As discussed above, PAGs of the invention are useful as the radiation sensitive component in photoresist compositions, including both positive-acting and negative-acting chemically amplified resist compositions.

The photoresists of the invention typically comprise a resin binder and a photoactive component of the invention as described above. Preferably the resin binder has functional groups that impart alkaline aqueous developability to the resist composition. For example, preferred are resin binders that comprise polar functional groups such as hydroxyl or carboxylate. Preferably the resin binder is used in a resist composition in an amount sufficient to render the resist developable with an aqueous alkaline solution.

For imaging at wavelengths greater than 200 nm, such as 248 nm, phenolic resins are typically preferred. Preferred phenolic resins are poly (vinylphenols) which may be formed by block polymerization, emulsion polymerization or solution polymerization of the corresponding monomers in the presence of a catalyst. Vinylphenols useful for the production of polyvinyl phenol resins may be prepared, for example, by hydrolysis of commercially available coumarin or substituted coumarin, followed by decarboxylation of the resulting hydroxy cinnamic acids. Useful vinylphenols may also be prepared by dehydration of the corresponding hydroxy alkyl phenols or by decarboxylation of hydroxy cinnamic acids resulting from the reaction of substituted or nonsubstituted hydroxybenzaldehydes with malonic acid. Preferred polyvinylphenol resins prepared from such vinylphenols have a molecular weight range of from about 2,000 to about 60,000 daltons.

Copolymers containing phenol and nonaromatic cyclic alcohol units also are preferred resin binders for resists of the invention and may be suitably prepared by partial hydrogenation of a novolak or poly(vinylphenol) resin. Such copolymers and the use thereof in photoresist compositions are disclosed in U.S. Patent No. 5,128,232 to Thackeray et al.

Additional suitable resins include those formed from bishydroxymethylated compounds, and block novolak resins. See U.S. Patents Nos. 5,130,410 and 5,128,230 where such resins and use of same in photoresist compositions is disclosed. Additionally, two or more resin binders of similar or different compositions can be blended or combined together to give additive control of lithographic properties of a photoresist composition. For instance, blends of resins can be used to adjust photospeed and thermal properties and to control dissolution behavior of a resist in a developer.

Preferably, a photoacid generator compound of the invention is employed in a chemically amplified positive-acting resist. A number of such resist compositions have been described, e.g., in U.S. Patents Nos. 4,968,581; 4,883,740; 4,810,613 and 4,491,628 and Canadian Patent Application 2,001,384, all of which are incorporated herein by reference for their teaching of making and using chemically amplified positive-acting resists. In accordance with the present invention, those prior resist compositions are modified by substitution of the photoactive component of the invention as the radiation sensitive component.

For imaging at wavelengths greater than 200 nm, such as 248 nm, a particularly preferred chemically amplified photoresist of the invention comprises in admixture a photoactive component of the invention and a resin binder that comprises a copolymer containing both phenolic and non-phenolic units. For example, one preferred group of such copolymers has acid labile groups substantially, essentially or completely only on non-phenolic units of the copolymer, particularly alkylacrylate photoacid-labile groups, i.e. a phenolic-alkyl acrylate copolymer. One especially preferred copolymer binder has repeating units x and y of the following formula: 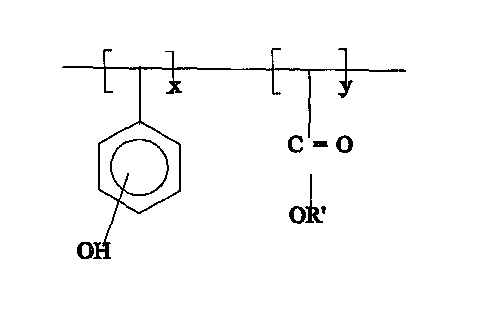 wherein the hydroxyl group be present at either the ortho, meta or para positions throughout the copolymer, and R' is substituted or unsubstituted alkyl having 1 to about 18 carbon atoms, more typically 1 to about 6 to 8 carbon atoms. *Tert*-butyl is a generally preferred R' group. An R' group may be optionally substituted by e.g. one or more halogen (particularly F, Cl or Br), C₁₋₈alkoxy, C₂₋₈alkenyl, etc. The units x and y may be regularly alternating in the copolymer, or may be randomly interspersed through the polymer. Such copolymers can be readily formed. For example, for resins of the above formula, vinyl phenols and a substituted or unsubstituted alkyl acrylate such as *t*-butylacrylate and the like may be condensed under free radical conditions as known in the art. The substituted ester moiety, i.e. R'-O-C(=O)-, moiety of the acrylate units serves as the acid labile groups of the resin and will undergo photoacid induced cleavage upon exposure of a coating layer of a photoresist containing the resin. Preferably the copolymer will have a M_{w} of from about 8,000 to about 50,000, more preferably about 15,000 to about 30,000 with a molecular weight distribution of about 3 or less, more preferably a molecular weight distribution of about 2 or less. Non-phenolic resins, e.g. a copolymer of an alkyl acrylate such as *t*-butylacrylate or t-butylmethacrylate and a vinyl alicyclic such as a vinyl norbornanyl or vinyl cyclohexanol compound, also may be used as a resin binder in compositions of the invention. Such copolymers also may be prepared by such free radical polymerization or other known procedures and suitably will have a M_{w} of from about 8,000 to about 50,000, and a molecular weight distribution of about 3 or less.

Other preferred resins that have acid-labile deblocking groups for use in a positive-acting chemically-amplified photoresist of the invention have been disclosed in European Patent Application 0829766A2 of the Shipley Company (resins with acetal and ketal resins) and European Patent Application EP0783136A2 of the Shipley Company (terpolymers and other copolymers including units of 1) styrene; 2) hydroxystyrene; and 3) acid labile groups, particularly alkyl acrylate acid labile groups such as t-butylacrylate or t-butylmethacrylate). In general, resins having a variety of acid labile groups will be suitable, such as acid sensitive esters, carbonates, ethers, imides, etc. The photoacid labile groups will more typically be pendant from a polymer backbone, although resins that have acid labile groups that are integral to the polymer backbone also may be employed.

PAGs of the invention (which includes PAGs of Formulae I, IA, IB, II, IIA, IIB, III, IIIA, IIIB, IV, IVA, IVB, V, VA, VI, and VIA as defined above) also are preferably used with polymers that contain one or more photoacid-labile groups and that are substantially, essentially or completely free of phenyl or other aromatic groups. Such photoresist compositions are particularly useful for imaging with sub-200 nm radiation such as 193 nm radiation.

For example, preferred polymers contain less than about 5 mole percent aromatic groups, more preferably less than about 1 or 2 mole percent aromatic groups, more preferably less than about 0.1, 0.02, 0.04 and 0.08 mole percent aromatic groups and still more preferably less than about 0.01 mole percent aromatic groups. Particularly preferred polymers are completely free of aromatic groups. Aromatic groups can be highly absorbing of sub-200 nm radiation and thus are undesirable for polymers used in photoresists imaged with such short wavelength radiation.

Suitable polymers that are substantially or completely free of aromatic groups and may be formulated with a PAG of the invention to provide a photoresist for sub-200 nm imaging are disclosed in European application EP930542A1 of the Shipley Company.

Suitable polymers that are substantially or completely free of aromatic groups suitably contain acrylate units such as photoacid-labile acrylate units as may be provided by polymerization of methyladamanatylacrylate, methyladamantylmethacrylate, ethylfenchylacrylate, ethylfenchylmethacrylate, and the like; fused non-aromatic alicyclic groups such as may be provided by polymerization of a norbornene compound or other alicyclic compound having an endocyclic carbon-carbon double bond; an anhydride such as may be provided by polymerization of maleic anhydride and/or itaconic anhydride; and the like.

Preferred negative-acting compositions of the invention comprise a mixture of materials that will cure, crosslink or harden upon exposure to acid, and a photoactive component of the invention.

Particularly preferred negative acting compositions comprise a resin binder such as a phenolic resin, a crosslinker component and a photoactive component of the invention. Such compositions and the use thereof has been disclosed in European Patent Applications 0164248 and 0232972 and in U.S. Patent No. 5,128,232 to Thackeray et al. Preferred phenolic resins for use as the resin binder component include novolaks and poly(vinylphenol)s such as those discussed above. Preferred crosslinkers include amine-based materials, including melamine, glycolurils, benzoguanamine-based materials and urea-based materials. Melamine-formaldehyde resins are generally most preferred. Such crosslinkers are commercially available, e.g. the melamine resins sold by American Cyanamid under the trade names Cymel 300, 301 and 303. Glycoluril resins are sold by American Cyanamid under trade names Cymel 1170, 1171, 1172, urea-based resins are sold under the trade names of Beetle 60, 65 and 80, and benzoguanamine resins are sold under the trade names Cymel 1123 and 1125.

Photoresists of the invention also may contain other materials. For example, other optional additives include actinic and contrast dyes, anti-striation agents, plasticizers, speed enhancers, sensitizers (e.g. for use of a PAG of the invention at longer wavelengths such as I-line (i.e. 365 nm) or G-line wavelengths), etc. Such optional additives typically will be present in minor concentration in a photoresist composition except for fillers and dyes which may be present in relatively large concentrations such as, e.g., in amounts of from 5 to 30 percent by weight of the total weight of a resist's dry components.

A preferred optional additive of resists of the invention is an added base, e.g. a caprolactam, which can enhance resolution of a developed resist relief image. The added base is suitably used in relatively small amounts, e.g. about 1 to 10 percent by weight relative to the PAG, more typically 1 to about 5 weight percent. Other suitable basic additives include ammonium sulfonate salts such as piperidinium p-toluenesulfonate and dicyclohexylammonium p-toluenesulfonate; alkyl amines such as tripropylamine and dodecylamine; aryl amines such as diphenylamine, triphenylamine, aminophenol, 2-(4-aminophenol)-2-(4-hydroxyphenyl)propane, etc.

The resin binder component of resists of the invention are typically used in an amount sufficient to render an exposed coating layer of the resist developable such as with an aqueous alkaline solution. More particularly, a resin binder will suitably comprise 50 to about 90 weight percent of total solids of the resist. The photoactive component should be present in an amount sufficient to enable generation of a latent image in a coating layer of the resist. More specifically, the photoactive component will suitably be present in an amount of from about 1 to 40 weight percent of total solids of a resist. Typically, lesser amounts of the photoactive component will be suitable for chemically amplified resists.

The photoresists of the invention are generally prepared following known procedures with the exception that a PAG of the invention is substituted for prior photoactive compounds used in the formulation of such photoresists. For example, a resist of the invention can be prepared as a coating composition by dissolving the components of the photoresist in a suitable solvent such as, e.g., a glycol ether such as 2-methoxyethyl ether (diglyme), ethylene glycol monomethyl ether, propylene glycol monomethyl ether; propylene glycol monomethyl ether acetate; lactates such as ethyl lactate or methyl lactate, with ethyl lactate being preferred; propionates, particularly methyl propionate, ethyl propionate and ethyl ethoxy propionate; a Cellosolve ester such as methyl Cellosolve acetate; an aromatic hydrocarbon such toluene or xylene; or a ketone such as methylethyl ketone, cyclohexanone and 2-heptanone. Typically the solids content of the photoresist varies between 5 and 35 percent by weight of the total weight of the photoresist composition. Blends of the such solvents also are suitable.

A particularly preferred photoresist of the invention contains the following components:
1) a resin component that contains fused cyclic units with a hetero ring member particularly oxygen (but other than an anhydride) such as may be provided by polymerized of a dihydropyran, preferably with one or more other repeat units particularly where one of the units contains a photoacid labile group units and preferably one of the units is an acrylate, especially a tetrapolymer of polymerized units of methyladamantylmethacrylate, maleic anhydride, norbornene; and 3,4-dihydro-*2H*-pyran, particularly a tetrapolymer containing, based on total polymer units, 40 mole percent of polymerized methyladamantylmethacrylate units, 30 mole percent of polymerized maleic anhydride, 10 mole percent of polymerized norbornene; and 20 mole percent of polymerized 3,4-dihydro-2*H*-pyran units;
2) a cyclic sulfonium PAG without phenyl substitution particularly para-tert-butylphenyl cyclotetramethylenesulfonium ((CH₂)₄>S⁺C₆H₄C(CH₃)₃) with a suitable counter anion such as perflorobutanesulfonate;
3) optionally, but preferably included, an added base particularly caprolactam;
4) optionally, but preferably included, a surfactant such as a fluorinated surfactant sold under the tradename of RO8 by Dai Nippon Ink;
5) a solvent component such as 2-heptanone or cyclohexanone e.g. as a single solvent as well as blended with other solvents such as ethyl lactate and propylene glycol methyl ether acetate, with 2-heptanone being a preferred solvent.

A further preferred photoresist of the invention contains the following components:
1) a resin component that contains fused cyclic units with a hetero ring member particularly oxygen (but other than an anhydride) such as may be provided by polymerized of a dihydropyran, preferably with one or more other repeat units particularly where one of the units contains a photoacid labile group units and preferably one of the units is an acrylate, especially a tetrapolymer of polymerized units of methyladamantylmethacrylate, maleic anhydride, norbornene; and 3,4-dihydro-*2H*-pyran, particularly a tetrapolymer containing, based on total polymer units, 40 mole percent of polymerized methyladamantylmethacrylate units, 30 mole percent of polymerized maleic anhydride, 10 mole percent of polymerized norbornene; and 20 mole percent of polymerized 3,4-dihydro-*2H*-pyran units;
2) one or more sulfonium PAGS such as a triphenylsulfonium ((C₆H₅)₃S⁺) triflate alone or in combination with triphenylsulfonium perfluorobutane;
3) optionally, but preferably included, an added base particularly caprolactam;
4) optionally, but preferably included, a surfactant such as a fluorinated surfactant sold under the tradename of RO8 by Dai Nippon Ink;
5) a solvent component such as cyclohexane or 2-heptanone e.g. as a single solvent as well as blended with other solvents such as ethyl lactate and propylene glycol methyl ether acetate, with cyclohexanone being a preferred solvent.

An especially preferred resin for use in the above preferred photoresists contains 40 mole percent (based on total resin units) polymerized methyladamantylmethacrylate groups; 30 mole percent (based on total resin units) polymerized maleic anhydride groups; 10 mole percent (based on total resin units) polymerized norbornene groups; and 20 mole percent (based on total resin units) of 3,4-dihydro-2*H*-pyran groups.

The photoresists of the invention can be used in accordance with known procedures. Though the photoresists of the invention may be applied as a dry film, they are preferably applied on a substrate as a liquid coating composition, dried by heating to remove solvent preferably until the coating layer is tack free, exposed through a photomask to activating radiation, optionally post-exposure baked to create or enhance solubility differences between exposed and nonexposed regions of the resist coating layer, and then developed preferably with an aqueous alkaline developer to form a relief image.

The substrate on which a resist of the invention is applied and processed suitably can be any substrate used in processes involving photoresists such as a microelectronic wafer. For example, the substrate can be a silicon, silicon dioxide or aluminum-aluminum oxide microelectronic wafer. Gallium arsenide, ceramic, quartz or copper substrates may also be employed. Printed circuit board substrates such as copper clad laminates are also particularly preferred. The photoresists of the invention will be particularly useful for circuit board imaging, including through hole and other aperture plating. Typical printed circuit board substrates have one or more copper layers interleaved with resin layers, such as epoxy layers.

Substrates used for liquid crystal display and other flat panel display applications are also suitably employed, e.g. glass substrates, indium tin oxide coated substrates and the like.

A liquid coating resist composition may be applied by any standard means such as spinning, dipping or roller coating. Photoresists of the invention also may be formulated and applied as dry film resists, particularly for printed circuit board manufacture applications. The exposure energy should be sufficient to effectively activate the photoactive component of the radiation sensitive system to produce a patterned image in the resist coating layer. Suitable exposure energies typically range from about 1 to 300 mJ/cm². As discussed above, preferred exposure wavelengths include sub-300 nm such as 248 nm, and sub-200 nm such as 193 nm and 157 nm. Suitable post-exposure bake temperatures are from about 50°C or greater, more specifically from about 50 to 140°C. For an acid-hardening negative-acting resist, a post-development bake may be employed if desired at temperatures of from about 100 to 150°C for several minutes or longer to further cure the relief image formed upon development. After development and any post-development cure, the substrate surface bared by development may then be selectively processed, for example chemically etching or plating substrate areas bared of photoresist in accordance with procedures known in the art. Suitable etchants include a hydrofluoric acid etching solution and a plasma gas etch such as an oxygen plasma etch.

All documents mentioned herein are incorporated herein by reference. The following non-limiting examples are illustrative of the invention.

### Example 1: Synthesis Using Phosphorous Pentoxide - Methanesulfonic Acid Condensation Method; synthesis of 2,3,4-trimethoxyphenyltetramethylenesulfonium triflate.

To a suspension of phosphorus oxide (6.0g, 0.021mol) and methanesulfonic acid (60.0g, 0.62mol), 1,2,3-trimethoxybenzene (17.2g, 0.10 mol) and tetramethylenesulfoxide (10.9g, 0.10mol) were added and reacted with stirring at 50°C for 3 hours. After the reaction, the reaction mixture was poured into H₂O (400ml), triflic acid (15.0g, 0.10mol) was added dropwise at 5°C and the reaction mixture stirred at room temperature overnight. Then, the aqueous mixture was washed with ether/hexane (100ml/100ml) and extracted with three 200ml portions of dichloromethane. The organic layer was washed with 1% aqueous ammonia (2 x 100ml) then H₂O (3 x 100ml), and concentrated in vacuo. The residue was crystallized with hexane and recrystallized from ethanol/ *t*-butylmethylether to give 30.0g (73%) of 2,3,4-trimethoxyphenyltetramethylenesulfonium triflate as a white powder.

Using appropriate reagents, the following compounds were prepared in the same manner as described immediately above for the synthesis of 2,3,4-trimethoxyphenyltetramethylene sulfonium triflate in this Example 1:
2,3,4-trimethoxyphenylpentamethylenesulfonium triflate;
4-methoxyphenyltetramethylene-sulfonium triflate; and
4-methoxyphenylpentamethylenesulfonium triflate.

### Example 2: Synthesis of 2,3,4-trimethoxyphenyltetramethylenesulfonium perfluoro-1-octanesulfonate.

To a suspension of phosphorus oxide (6.0g, 0.021mol) and methanesulfonic acid (60.0g, 0.62mol), 1,2,3-trimethoxybenzene (17.2g, 0.10mol) and tetramethylenesulfoxide (10.9g, 0.10mol) were added and reacted with stirring at 50°C for 3 hours. After the reaction, the reaction mixture was poured into H₂O (400ml) and perfluoro-1-octanesulfonic acid (50.0g, 0.10mol) was added at 5°C. The sulfonium salt was precipitated, filtered and dissolved in dichloromethane (600ml). The organic layer was washed with 1% aqueous ammonia (2 x 100ml), then H₂O (3 x 100ml), and concentrated in vacuo. The residue was crystallized from hexane to give 51.1 g (67%) of 2,3,4-trimethoxyphenyltetramethylenesulfonium perfluoro-1-octanesulfonate as a white powder.

Using appropriate reagents, the following compounds were compounds were prepared as described immediately above for 2,3,4-trimethoxyphenyltetramethylene sulfonium perfluoro-1-octanesulfonate in this Example 2:
2,3,4-trimethoxyphenylpentamethylenesulfonium perfluoro-l-octanesulfonate;
4-methoxyphenyl-tetramethylenesulfonium perfluoro-1-octanesulfonate; and
4-methoxyphenylpentamethylene-sulfonium perfluoro-l-octanesulfonate.

### Example 3: Synthesis Using S-phenylation via iodonium salt method; synthesis of 4-t-butylphenyltetramethylenesulfonium triflate

To a mixture of bis-(4-t-butylphenyl)iodonium triflate (20.0g, 37mmol) and tetrahydothiophene (3.3g, 37mmol) in chlorobenzene (30ml), copper (II) benzoate (0.3g, 0.95mmol) was added as catalyst and reacted with stirring at 120°C for 3 hours. After cooling, the sulfonium salt was precipitated, washed with ether (200ml), filtered and then dissolved in dichloromethane (300ml). The organic layer was washed with 1% aqueous ammonia (3 x 50ml) then H₂O (3 x 50ml), and concentrated in vacuo. The residual solid was purified with *t*-butylmethylether to give 9.5g (69%) of 4-t-butylphenyltetramethylenesulfonium triflate as a white powder.

Using appropriate reagents, the following compounds were prepared in the same manners as described immediately above for 4-t-butylphenyltetramethylene sulfonium triflate in this Example 3:
4-t-Butylphenyltetramethylenesulfonium perfluoro-l-butanesulfonate;
4-t-butylphenyl-tetramethylenesulfonium perfluoro-1-octanesulfonate;
4-t-butylphenyltetramethylenesulfonium trifluoromethanesulfonimide;
4-t-butylphenylpentamethylenesulfonium perfluoro-1-butanesulfonate;
4-t-butylphenylpentamethylenesulfonium perfluoro-l-octanesulfonate; and
4-t-butylphenyl-pentamethylenesulfonium trifluoromethanesulfonimide.

### Example 4: Synthesis of Bis-Cyclic Sulfonium PAGs via Double Cyclization Method; preparation of phenyl 1,4-di-(pentamethylenesulfonium perfluoro-l-butanesulfonate)

To a solution of 1,4-dibenzenethiol [1.89g (13.3mmol)] in methanol (10ml), 25wt% sodium methoxide in methanol [5.74g (26.6mmol)] was added and stirred for 10 minutes at room temperature. 5-Chloro-1-pentanol [5.14g (39.9mmol)] was then added dropwise to the solution. After 3 hours at reflux, 10ml of water was added and the solvent was evaporated. The residue was extracted with dichloromethane (150ml) and the solvent was evaporated. The resulting white solid was recrystallized from acetonitrile and dried to give phenyl 1,4-di-(5-hydroxypentyl)sulfide in 3.26g (78%). Sodium iodide [3.42g (22.8mmol)] was added to 120ml of acetonitrile solution of the phenyl 1,4-di-(5-hydroxypentyl)sulfide, followed by dropwise addition of chlorotrimethylsilane [2.48g (22.8mmol)], and then refluxing the mixture for 15 hours. After cooling to room temperature, a few drops of aqueous 10wt% ammonium chloride were added, followed by dichloromethane extraction. The organic layer was sequentially washed with aqueous 20wt% sodium thiosulfate and saturated ammonium chloride, dried, and evaporated to give phenyl 1,4-di-(5-iodopentyl)sulfide in 9.18g (99%).

Silver perfluoro-l-butanesulfonate [14.77g (36.28mmol)] was added to 500ml of acetonitrile solution of the phenyl 1,4-di-(5-iodopentyl)sulfide and the resulting mixture stirred for 18 hours at room temperature. After stirring, the precipitate (silver iodide) was filtered out and the filtrate was concentrated. The residue was dissolved in 500ml of ethanol and filtered, and then the filtrate was evaporated. The obtained solids were washed with ether, aqueous ammonia and water, and then recrystallized from mixture of ethanol and ether to give pure phenyl 1,4-di-(pentamethylenesulfonium perfluoro-1-butanesulfonate) (8.30g, 55%).

### Example 5: Resist preparation and lithographic processing.

Resist formulations were prepared by dissolving polymers, poly[4-hydroxystyrene/styrene/2-methyl-2-adamantane methacrylate], (11 to 13 wt % based on total solids) in a mixture of ethyl lactate and propylene-glycol methylether acetate, with photoacid generator compounds as specified below (5 to 7 wt. % vs. polymer weight), base additives (0.2 to 0.5 wt% vs. polymer weight) and surfactant (0.05-0.10 wt% vs. total solids). The resist solutions were filtered through 0.2 µm Teflon membrane filters prior to use.

Three separate photoresists (designated as Resists A, B and C respectively) were prepared. Those resists differed only in the type (but not the amount) of the photoacid generator component. Resist A contained 4-t-butylphenylcyclotetramethylenesulfonium (Compound 1 above). Resist B contained 4-t-butylphenylcyclopentamethylenesulfonium (Compound 2 above) PAG. Resist C (comparative) contained triphenylsulfonium PAG. For each of the Resists A, B and C, the PAGs were present with a mixture of anions of triflate and perfluoroctanesulfonate, i.e. Resist A contained a mixture of 4-t-butylphenylcyclotetramethylenesulfonium triflate and 4-t-butylphenylcyclotetramethylenesulfonium perfluorooctanesulfonate, etc.

Resist solutions were spin-coated over an organic anti-reflective coating on 8 inch silicon wafers to provide approximately 4150 Å thick resist coating layers. The resist layers were softbaked at 130°C for 60 seconds, exposed to 248 nm radiation using 0.63 NA, annular illumination. The exposed resist later was then baked at 130°C for 90 seconds and developed with an aqueous alkaline developer for 30 seconds.

Resists A and B exhibited enhanced resist profile and process window relative to comparative Resist C.

The foregoing description of the invention is merely illustrative thereof, and it is understood that variations and modifications can be effected without departing from the spirit or scope of the invention as set forth in the following claims.

## Claims

1. A photoresist composition comprising a resin and a photoacid generator compound of the following Formula I: 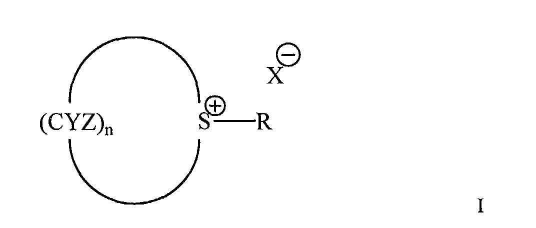 wherein R is a non-hydrogen substituent or R is a linkage to another photoacid generator moiety;
Y and Z are each independently hydrogen or a non-hydrogen substituents;
or two of Y and/or Z are taken together to form a fused ring;
n is an integer of from 3 to 8; and X is a counter anion,
with the exclusion of Y and Z both being C₁₋₄alkyl on the same ring carbon.

2. A photoresist composition comprising a resin and a photoacid generator compound of the following Formula I: 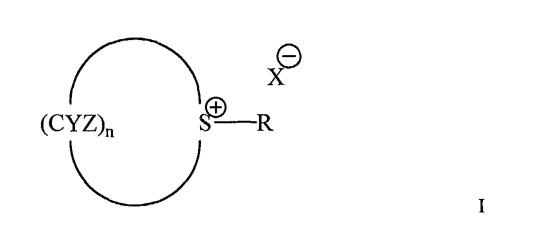 wherein R is a non-hydrogen substituent or R is a linkage to another photoacid generator moiety;
Y and Z are each independently hydrogen or a non-hydrogen substituents;
or two of Y and/or Z are taken together to form a fused ring;
n is an integer of from 3 to 8; and X is a counter anion,
with the exclusion of R, when being other than a linkage to another photoacid generator moiety, being unsubstituted phenyl, phenyl substituted by hydroxy or alkoxy, unsubstituted naphthyl, or naphthyl substituted by hydroxy or alkoxy.

3. A photoresist composition of claim 1 or 2 wherein the photoacid generator compound is of the following Formula IA: 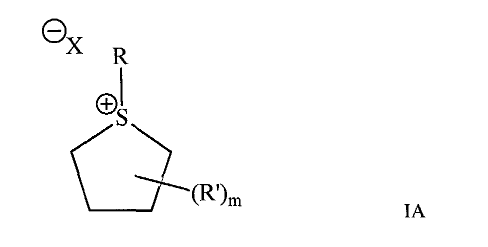 wherein R is a non-hydrogen substituent or a linkage to another photoacid generator moiety;
R' is a non-hydrogen substituents;
m is an integer of from 0 to 8; and X is a counter anion.

4. A photoresist composition of claim 1 or 2 wherein the photoacid generator compound is of the following Formula IB: 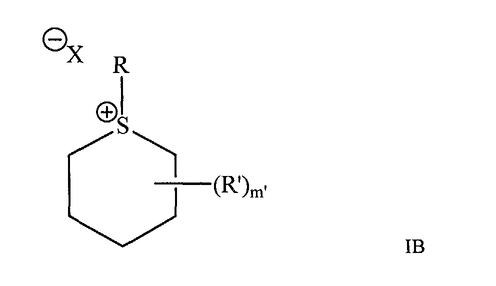 wherein R is a non-hydrogen substituent or a linkage to another photoacid generator moiety;
R' is a non-hydrogen substituents;
m' is an integer of from 0 to 10; and X is a counter anion.

5. A photoresist composition of claim 1 wherein the photoacid generator compound is of the following Formula II: 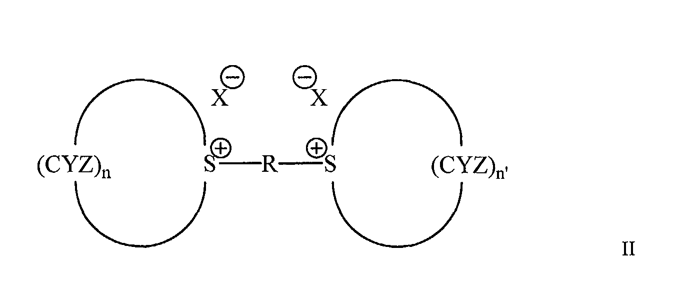 wherein R is a chemical bond or a linker;
each Y and each Z are independently hydrogen or a non-hydrogen substituent;
n and n' are each independently an integer of from 3 to 8; and each X is the same or different counter anion.

6. A photoresist composition of claim 1 comprising a resin and a photoacid generator compound of the following Formula IIA: 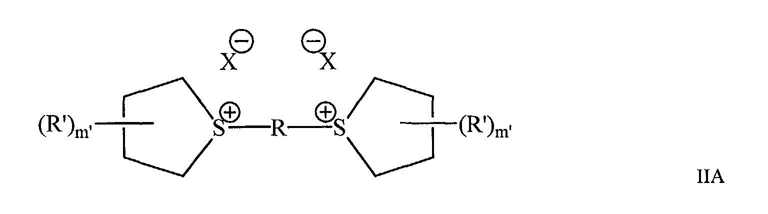 wherein R is a chemical bond or a linker; each R' is the same or different non-hydrogen substituent; each m is independently an integer of from 0 to 8; and X is a counter anion.

7. A photoresist composition comprising a resin and a photoacid generator compound of the following Formula IIB: 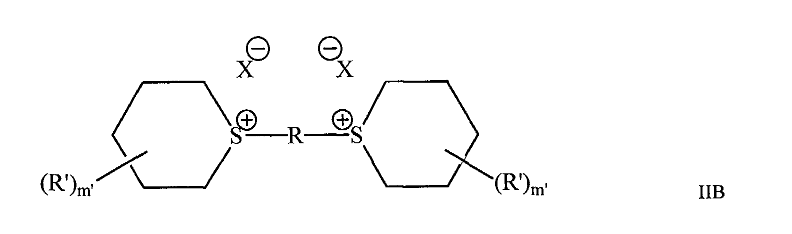 wherein R is a chemical bond or a linker; each R¹ is the same or different non-hydrogen substituent; each m' is independently an integer of from 0 to 8; and X is a counter anion.

8. A photoresist composition comprising a resin and a photoacid generator compound of the following Formula III: 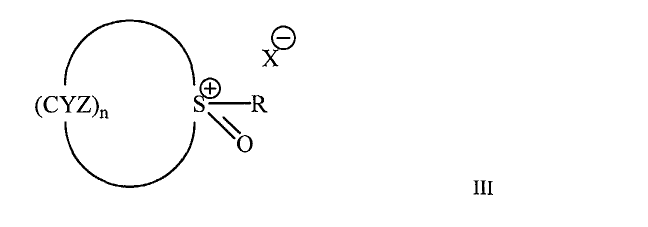 wherein R is a non-hydrogen substituent or R is a linkage to another photoacid generator moiety; Y and Z are each independently hydrogen or a non-hydrogen substituents, or two of Y and/or Z are taken together to form a fused ring; n is an integer of from 3 to 8; and X is a counter anion.

9. A photoresist composition of claim 8 wherein the photoacid generator compound is of the following Formula IIIA 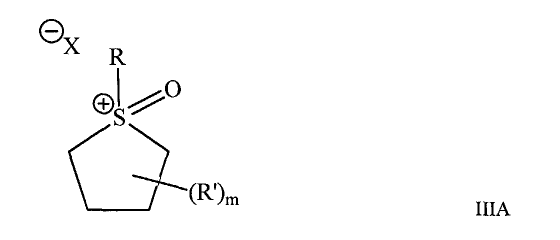 wherein R is a chemical bond or a linker; each R¹ is the same or different non-hydrogen substituent; m is an integer of from 0 to 8; and X is a counter anion.

10. A photoresist composition of claim 8 wherein the photoacid generator compound is of the following Formula IIIB: 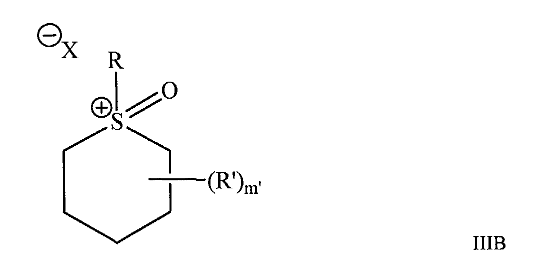 wherein R is a chemical bond or a linker; each R¹ is the same or different non-hydrogen substituent; m' is an integer of from 0 to 8; and X is a counter anion.

11. A photoresist composition of claim 8 wherein the photoacid generator compound is of the following Formula IV: 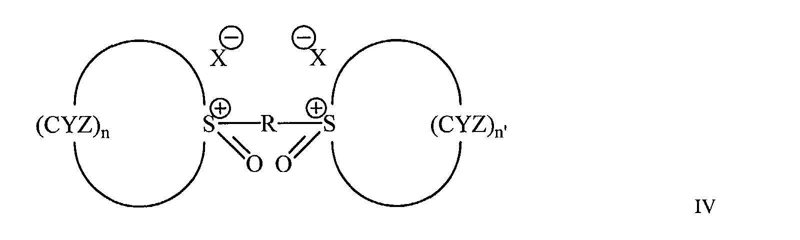 wherein R is a linkage to another photoacid generator moiety; Y and Z are each independently hydrogen or a non-hydrogen substituents, or two of Y and/or Z are taken together to form a fused ring; n and n' are each independently a linkage of from 3 to 8; and X is a counter anion.

12. A photoresist composition of claim 1 wherein the photoacid generator is of the following Formula V: 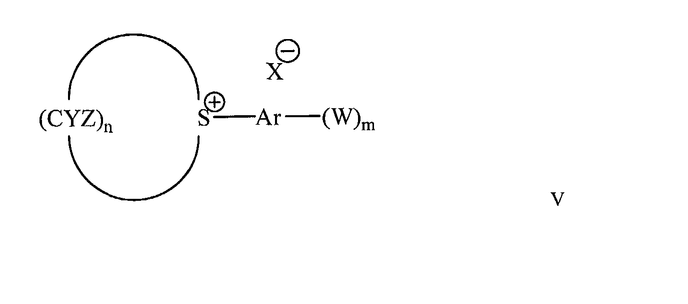 wherein Y and Z are each independently hydrogen or a non-hydrogen substituents, or two of Y and/or Z are taken together to form a fused ring; n and n' are each independently a linkage of from 3 to 8;
Ar is an optionally substituted aryl group,
each W is an optionally substituted alkoxy or photoacid-labile ring substituent of the Ar group; and m is an integer of from 1 to 5.

13. A photoresist composition of claim 1 wherein the photoacid generator is of the following Formula VI: 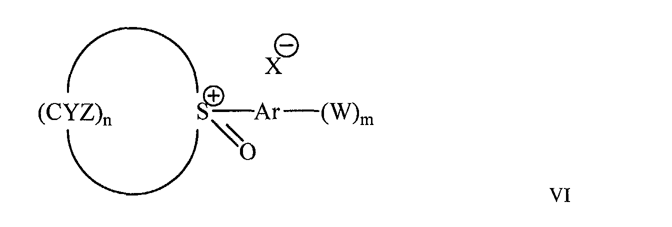 wherein Y and Z are each independently hydrogen or a non-hydrogen substituents, or two of Y and/or Z are taken together to form a fused ring; n and n' are each independently a linkage of from 3 to 8;
Ar is an optionally substituted aryl group,
each W is an optionally substituted alkoxy or photoacid-labile ring substituent of the Ar group; and m is an integer of from 1 to 5.

14. A photoresist composition of claim 1, 2, 3, 4, 8, 9 or 10 wherein R is optionally substituted alkyl, optionally substituted carbocyclic aryl, optionally substituted heteroaromatic, or optionally substituted heteroalicyclic.

15. A photoresist composition of claim 5, 6, 7 or 11 wherein R is a chemical bond, optionally substituted alkylene, or optionally substituted carbocyclic aryl.

16. A photoresist composition of any one of claims 1, 2, 5, 8, 11, 12 or 13 wherein each Y and each Z are independently optionally substituted carbocyclic aryl, optionally substituted heteroaromatic or optionally substituted heteroalicyclic.

17. A photoresist composition of any one of claims 1, 2, 5, 8, 11, 12 or 13 wherein two Y and Z groups are taken together to form a ring fused to the sulfonium or sulfoxonium ring.

18. A photoresist composition of claim 17 wherein the fused ring is an optionally substituted carbon alicyclic ring, optionally substituted carbocyclic aryl, optionally substituted heteroaromatic or optionally substituted heteroalicyclic.

19. A photoresist composition of claim 17 or 18 wherein the two R¹ groups taken together are substituents of adjacent carbon atoms of the sulfonium or sulfoxonium ring.

20. A photoresist composition of claim 17 or 18 wherein the two R¹ groups taken together are substituents of non-adjacent carbon atoms of the sulfonium or sulfoxonium ring.

21. A photoresist composition of any one of claims 3, 4, 6, 7, 9 or 10 wherein one or more R¹ groups are optionally substituted carbocyclic aryl, optionally substituted heteroaromatic or optionally substituted heteroalicyclic.

22. A photoresist composition of any one of claims 3, 4, 6, 7, 9 or 10 wherein two R¹ groups are taken together to form a ring fused to the sulfonium or sulfoxonium ring.

23. A photoresist composition of claim 22 wherein the fused ring is an optionally substituted carbon alicyclic ring, optionally substituted carbocyclic aryl, optionally substituted heteroaromatic or optionally substituted heteroalicyclic.

24. A photoresist composition of claim 22 or 23 wherein the two R¹ groups taken together are substituents of adjacent carbon atoms of the sulfonium or sulfoxonium ring.

25. A photoresist composition of claim 22 or 23 wherein the two R¹ groups taken together are substituents of non-adjacent carbon atoms of the sulfonium or sulfoxonium ring.

26. A photoresist composition comprising a resin and a multicyclic sulfonium photoacid generator compound.

27. The photoresist of claim 26 wherein the sulfonium compound is a bicyclic compound.

28. The photoresist of claim 26 wherein the sulfonium compound is a tricyclic compound.

29. The photoresist of any one of claims 26 through 28 wherein the sulfonium atom is a ring member of a five-atom ring.

30. The photoresist of any one of claims 26 through 28 wherein the sulfonium atom is a ring member of a six-atom ring.

31. A photoresist composition comprising a resin and a multicyclic sulfoxonium photoacid generator compound.

32. The photoresist of claim 31 wherein the sulfoxonium compound is a bicyclic compound.

33. The photoresist of claim 31 wherein the sulfoxonium compound is a tricyclic compound.

34. The photoresist of any one of claims 31 through 33 wherein the sulfoxonium atom is a ring member of a five-atom ring.

35. The photoresist of any one of claims 31 through 34 wherein the sulfoxonium atom is a ring member of a six-atom ring.

36. The photoresist composition of any one of claims 1 through 35 wherein the composition is a chemically-amplified positive-acting photoresist.

37. The photoresist composition of claim 36 wherein the resin comprises a polymer that contains phenolic and photoacid-labile alkyl acrylate units.

38. The photoresist composition of claim 36 wherein the resin comprises a polymer that contains 1) phenolic units, 2) phenyl units, and 3) photoacid-labile alkyl acrylate units.

39. The photoresist composition of claim 36 wherein the resin comprises acetal or ketal groups.

40. The photoresist composition of claim 36 wherein the photoresist is essentially free of polymers containing aromatic units.

41. The photoresist composition of any one of claims 36 through 40 wherein the photoresist comprises a polymer that comprises polymerized alicyclic olefin groups.

42. The photoresist composition of claim 41 wherein the photoresist comprises a polymer that comprises polymerized optionally substituted norbornene units.

43. The photoresist of any one of claims 36 through 42 wherein the photoresist comprises a polymer that comprises polymerized alkyl acrylate groups.

44. The photoresist composition of any one of claims 1 through 35 wherein the composition is a negative-acting photoresist.

45. The photoresist composition of any one of claims 1 through 35 wherein the photoresist comprise a resin having fluorine substitution.

46. A method for forming a photoresist relief image on a substrate comprising:
(a) applying a coating layer of a photoresist composition of any one of claims 1 through 45 on a substrate; and
(b) exposing the photoresist coating layer to patterned activating radiation and developing the exposed photoresist layer to provide a relief image.

47. The method of claim 46 wherein the photoresist coating layer is exposed to radiation having a wavelength of less than about 300 nm.

48. The method of claim 46 wherein the photoresist coating layer is exposed to radiation having a wavelength of about 248 nm.

49. The method of claim 46 wherein the photoresist coating layer is exposed to radiation having a wavelength of less than about 200 nm.

50. The method of claim 46 wherein the photoresist coating layer is exposed to radiation having a wavelength of about 193 nm or 157 nm.

51. An article of manufacture having on at least one surface a coating layer of the photoresist composition of any one of claims 1 through 45

52. The article of claim 51 wherein the photoresist composition is coated on a microelectronic wafer substrate, flat panel display substrate or a printed circuit board substrate.

53. A photoacid generator compound of the following Formula I: 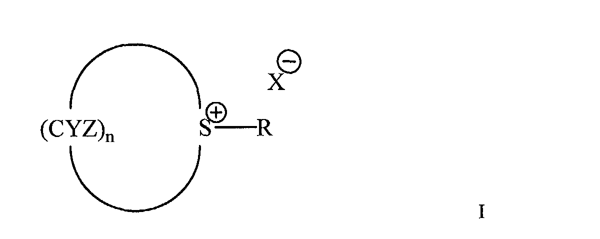 wherein R is a non-hydrogen substituent or R is a linkage to another photoacid generator moiety;
Y and Z are each independently hydrogen or a non-hydrogen substituents;
or two of Y and/or Z are taken together to form a fused ring;
n is an integer of from 3 to 8; and X is a counter anion,
with the exclusion of Y and Z both being C₁₋₄alkyl on the same ring carbon.

54. A photoacid generator compound of the following Formula I: 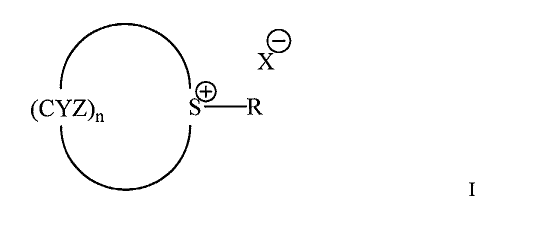 wherein R is a non-hydrogen substituent or R is a linkage to another photoacid generator moiety;
Y and Z are each independently hydrogen or a non-hydrogen substituents;
or two of Y and/or Z are taken together to form a fused ring;
n is an integer of from 3 to 8; and X is a counter anion,
with the exclusion of R, when being other than a linkage to another photoacid generator moiety, being unsubstituted phenyl, phenyl substituted by hydroxy or alkoxy, unsubstituted naphthyl, or naphthyl substituted by hydroxy or alkoxy.

55. A photoacid generator compound of claim 53 or 54 wherein the photoacid generator compound is of the following Formula IA: 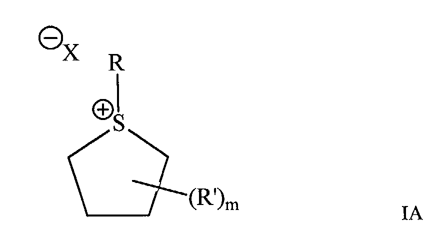 wherein R is a non-hydrogen substituent or a linkage to another photoacid generator moiety;
R¹ is a non-hydrogen substituents, with the exclusion of two R¹ groups both being present as C₁₋₄alkyl on the same ring carbon;
m is an integer of from 0 to 8; and X is a counter anion.

56. A photoacid generator compound of claim 53 or 54 wherein the photoacid generator compound is of the following Formula IB: 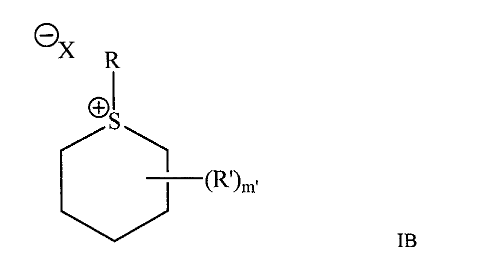 wherein R is a non-hydrogen substituent or a linkage to another photoacid generator moiety;
R¹ is a non-hydrogen substituents, with the exclusion of two R¹ groups both being present as C₁₋₄alkyl on the same ring carbon;
m' is an integer of from 0 to 10; and X is a counter anion.

57. A photoresist composition of claim 53 or 54 wherein the photoacid generator compound is of the following Formula II: 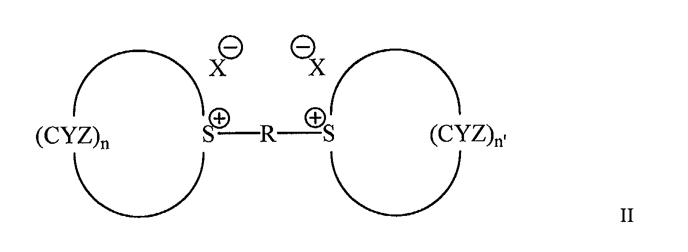 wherein R is a chemical bond or a linker;
each Y and each Z are independently hydrogen or a non-hydrogen substituent;
n and n' are each independently an integer of from 3 to 8; and each X is the same or different counter anion.

58. A photoacid generator compound of claim 53 or 54 wherein the photoacid generator compound is of the following Formula IIA: 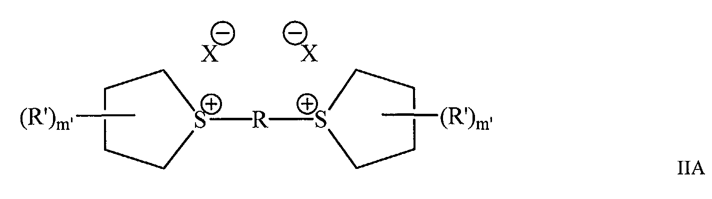 wherein R is a chemical bond or a linker; each R¹ is the same or different non-hydrogen substituent; each m is independently an integer of from 0 to 8; and X is a counter anion.

59. A photoacid generator compound of claim 53 or 54 wherein the photoacid generator compound is of the following Formula IIB: 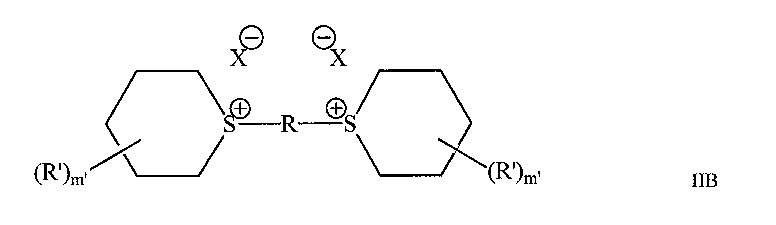 wherein R is a chemical bond or a linker; each R¹ is the same or different non-hydrogen substituent; each m' is independently an integer of from 0 to 10; and X is a counter anion.

60. A photoacid generator compound of the following Formula III: 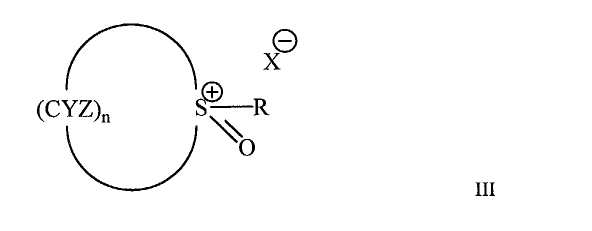 wherein R is a non-hydrogen substituent or R is a linkage to another photoacid generator moiety; Y and Z are each independently hydrogen or a non-hydrogen substituents, or two of Y and/or Z are taken together to form a fused ring; n is an integer of from 3 to 8; and X is a counter anion.

61. A photoacid generator compound of claim 60 wherein the photoacid generator compound is of the following Formula IIIA 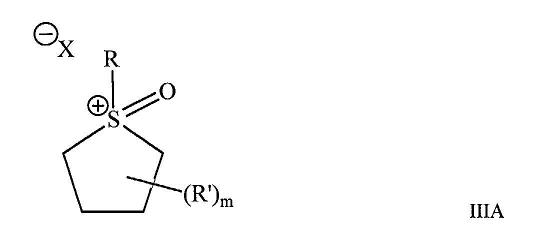 wherein R is a chemical bond or a linker; each R¹ is the same or different non-hydrogen substituent; m is an integer of from 0 to 8; and X is a counter anion.

62. A photoacid generator compound of claim 60 wherein the photoacid generator compound is of the following Formula IIIB: 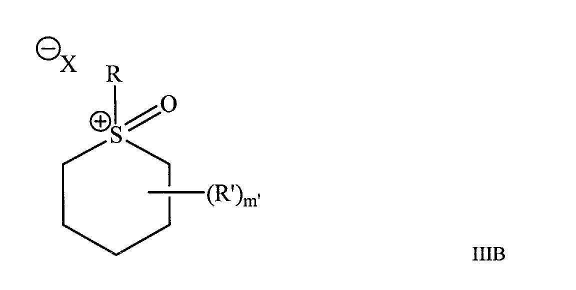 wherein R is a chemical bond or a linker; each R¹ is the same or different non-hydrogen substituent; m' is an integer of from 0 to 8; and X is a counter anion.

63. A photoresist composition of claim 60 wherein the photoacid generator compound is of the following Formula IV: 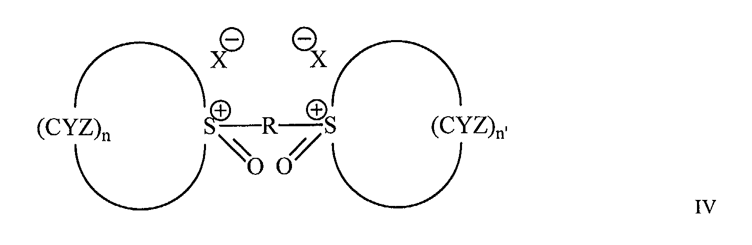 wherein R is a linkage to another photoacid generator moiety; Y and Z are each independently hydrogen or a non-hydrogen substituents, or two of Y and/or Z are taken together to form a fused ring; n and n' are each independently a linkage of from 3 to 8; and X is a counter anion.

64. A photoacid generator is of the following Formula V: 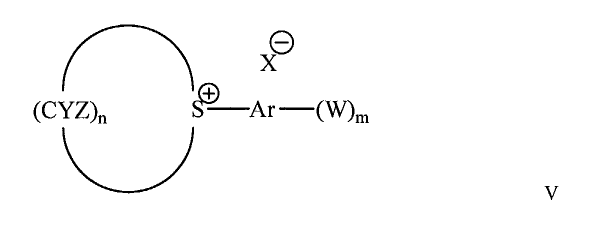 wherein Y and Z are each independently hydrogen or a non-hydrogen substituents, or two of Y and/or Z are taken together to form a fused ring; n and n' are each independently a linkage of from 3 to 8;
Ar is an optionally substituted aryl group,
each W is an optionally substituted alkoxy or photoacid-labile ring substituent of the Ar group; and m is an integer of from 1 to 5.

65. A photoacid generator is of the following Formula VI: 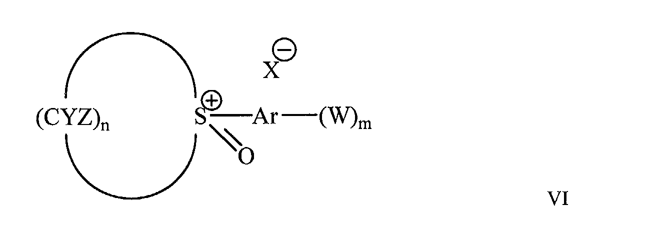 wherein Y and Z are each independently hydrogen or a non-hydrogen substituents, or two of Y and/or Z are taken together to form a fused ring; n and n' are each independently a linkage of from 3 to 8;
Ar is an optionally substituted aryl group,
each W is an optionally substituted alkoxy or photoacid-labile ring substituent of the Ar group; and m is an integer of from 1 to 5.

66. A photoresist composition of claim 53, 54, 55 or 57 wherein R is optionally substituted alkyl, optionally substituted carbocyclic aryl, optionally substituted heteroaromatic, or optionally substituted heteroalicyclic.

67. A photoresist composition of claim 53, 54, 55 or 57 wherein R is a chemical bond, optionally substituted alkylene, or optionally substituted carbocyclic aryl.

68. A photoresist composition of any one of claims 53, 54, 57, 60, 61, 64 or 65 wherein each Y and each Z are independently optionally substituted carbocyclic aryl, optionally substituted heteroaromatic or optionally substituted heteroalicyclic.

69. A photoresist composition of any one of claims 53, 54, 57, 60, 61, 64 or 65 wherein two Y and Z groups are taken together to form a ring fused to the sulfonium or sulfoxonium ring.

70. A photoresist composition of claim 69 wherein the fused ring is an optionally substituted carbon alicyclic ring, optionally substituted carbocyclic aryl, optionally substituted heteroaromatic or optionally substituted heteroalicyclic.

71. A photoresist composition of claim 69 or 70 wherein the two R¹ groups taken together are substituents of adjacent carbon atoms of the sulfonium or sulfoxonium ring.

72. A photoresist composition of claim 69 or 70 wherein the two R¹ groups taken together are substituents of non-adjacent carbon atoms of the sulfonium or sulfoxonium ring.

73. A photoresist composition of any one of claims 55, 56, 58, 59; 61 or 62 wherein one or more R¹ groups are optionally substituted carbocyclic aryl, optionally substituted heteroaromatic or optionally substituted heteroalicyclic.

74. A photoresist composition of any one of claims 55, 56, 58, 59, 61 or 62 wherein two R¹ groups are taken together to form a ring fused to the sulfonium or sulfoxonium ring.

75. A photoresist composition of claim 74 wherein the fused ring is an optionally substituted carbon alicyclic ring, optionally substituted carbocyclic aryl, optionally substituted heteroaromatic or optionally substituted heteroalicyclic.

76. A photoresist composition of claim 74 or 75 wherein the two R¹ groups taken together are substituents of adjacent carbon atoms of the sulfonium or sulfoxonium ring.

77. A photoresist composition of claim 74 or 75 wherein the two R¹ groups taken together are substituents of non-adjacent carbon atoms of the sulfonium or sulfoxonium ring.

78. A multicyclic sulfonium photoacid generator compound.

79. The sulfonium compound of claim 78 where the sulfonium compound is a bicyclic compound.

80. The sulfonium compound of claim 78 wherein the sulfonium compound is a tricyclic compound.

81. The sulfonium compound of claim of any one of claims 78 through 80 wherein the sulfonium atom is a ring member of a five-atom ring.

82. The sulfonium compound of any one of claims 78 through 80 wherein the sulfonium atom is a ring member of a six-atom ring.

83. A multicyclic sulfoxonium photoacid generator compound.

84. The sulfoxonium compound of claim 83 wherein the sulfoxonium compound is a bicyclic compound.

85. The sulfoxonium compound of claim 83 wherein the sulfoxonium compound is a tricyclic compound.

86. The sulfoxonium compound of any one of claims 83 through 85 wherein the sulfoxonium atom is a ring member of a five-atom ring.

87. The sulfoxonium compound of any one of claims 83 through 85 wherein the sulfoxonium atom is a ring member of a six-atom ring.
